# EUROPEAN PATENT APPLICATION

(11) **EP 1 946 765 A1**
(43) Date of publication of application: **23.07.2008**
(21) Application number: 06810198.9
(22) Date of filing: 15.09.2006
(51) Int. Cl.: A61K 38/00, A01K 67/027, A61K 31/7088, A61K 45/00, A61K 48/00, A61P 9/00, A61P 9/10, A61P 27/02, A61P 43/00, C12Q 1/02, C12Q 1/68, G01N 33/15, G01N 33/50, C12N 15/09

(54) **REMEDY FOR ANGIOGENESIS-RELATED DISEASE COMPRISING CHONDROMODULIN-I AS THE ACTIVE INGREDIENT**

(30) Priority: 22.09.2005 JP 2005275479
(71) Applicant: Fukuda, Keiichi, Tokyo 1760006 (JP); Hiraki, Yuji, Kawachinagano-shi Osaka-fu (JP)
(72) Inventor: Fukuda, Keiichi, Tokyo 1760006 (JP); Hiraki, Yuji, Kawachinagano-shi Osaka-fu (JP)
(74) Representative: Vossius & Partner
(86) International application number: PCT/JP2006/318406
(87) International publication number: WO 2007/034753

(57) **Abstract**

An objective of the present invention is to analyze the function of anti-angiogenic factors in cardiac valves or such to elucidate the developmental mechanism of angiogenesis-induced diseases. A more specific objective is to provide therapeutic agents for angiogenesis-induced diseases such as valvular heart disease, and methods of efficiently screening for the therapeutic agents. The present inventors discovered that chondromodulin-I was markedly expressed in cardiac valves, and plays an important role in maintaining normal functions of the valves by preventing angiogenesis, thickening, and calcification which lead to valvular heart diseases. Chondromodulin-I proteins and substances that activate the expression or function of the proteins are expected to have therapeutic effects against angiogenesis-induced diseases.

SEQUENCE LISTING

<110> FUKUDA, KEIICHI
      HIRAKI, YUJI

<120> REMEDY FOR ANGIOGENESIS-RELATED DISEASE COMPRISING CHONDROMODULIN-I AS THE
ACTIVE INGREDIENT

<130> C1-A0512P

<150> JP 2005-275479
<151> 2005-09-22

<160> 11

<170> PatentIn version 3.3

<210> 1
<211> 1005
<212> DNA
<213> Homo sapiens

<400> 1
atgacagaga actccgacaa agttcccatt gccctggtgg gacctgatga cgtggaattc      60

tgcagccccc cggcgtacgc tacgctgacg gtgaagccct ccagccccgc gcggctgctc     120

aaggtgggag ccgtggtcct catttcggga gctgtgctgc tgctctttgg ggccatcggg     180

gccttctact tctggaaggg gagcgacagt cacatttaca atgtccatta caccatgagt     240

atcaatggga aattacaaga tgggtcaatg gaaatagacg ctgggaacaa cttggagacc     300

tttaaaatgg gaagtggagc tgaagaagca attgcagtta atgatttcca gaatggcatc     360

acaggaattc gttttgctgg aggagagaag tgctacatta aagcgcaagt gaaggctcgt     420

attcctgagg tgggcgccgt gaccaaacag agcatctcct ccaaactgga aggcaagatc 480

atgccagtca aatatgaaga aaattctctt atctgggtgg ctgtagatca gcctgtgaag 540

gacaacagct tcttgagttc taaggtgtta gaactctgcg gtgaccttcc tattttctgg 600

cttaaaccaa cctatccaaa agaaatccag agggaaagaa gagaagtggt aagaaaaatt 660

gttccaacta ccacaaaaag accacacagt ggaccacgga gcaacccagg cgctggaaga 720

ctgaataatg aaaccagacc cagtgttcaa gaggactcac aagccttcaa tcctgataat 780

ccttatcatc agcaggaagg ggaaagcatg acattcgacc ctagactgga tcacgaagga 840

atctgttgta tagaatgtag gcggagctac acccactgcc agaagatctg tgaacccctg 900

gggggctatt acccatggcc ttataattat caaggctgcc gttcggcctg cagagtcatc 960

atgccatgta gctggtgggt ggcccgtatc ttgggcatgg tgtga 1005

<210> 2
<211> 334
<212> PRT
<213> Homo sapiens

<400> 2

Met Thr Glu Asn Ser Asp Lys Val Pro Ile Ala Leu Val Gly Pro Asp
1               5                   10                  15

Asp Val Glu Phe Cys Ser Pro Pro Ala Tyr Ala Thr Leu Thr Val Lys
            20                  25                  30

Pro Ser Ser Pro Ala Arg Leu Leu Lys Val Gly Ala Val Val Leu Ile
        35                  40                  45

Ser Gly Ala Val Leu Leu Leu Phe Gly Ala Ile Gly Ala Phe Tyr Phe
    50                  55                  60

Trp Lys Gly Ser Asp Ser His Ile Tyr Asn Val His Tyr Thr Met Ser
65                  70                  75                  80

Ile Asn Gly Lys Leu Gln Asp Gly Ser Met Glu Ile Asp Ala Gly Asn
                85                  90                  95

Asn Leu Glu Thr Phe Lys Met Gly Ser Gly Ala Glu Glu Ala Ile Ala
            100                 105                 110

Val Asn Asp Phe Gln Asn Gly Ile Thr Gly Ile Arg Phe Ala Gly Gly
        115                 120                 125

Glu Lys Cys Tyr Ile Lys Ala Gln Val Lys Ala Arg Ile Pro Glu Val
    130                 135                 140

Gly Ala Val Thr Lys Gln Ser Ile Ser Ser Lys Leu Glu Gly Lys Ile
145                 150                 155                 160

Met Pro Val Lys Tyr Glu Glu Asn Ser Leu Ile Trp Val Ala Val Asp
                165                 170                 175

Gln Pro Val Lys Asp Asn Ser Phe Leu Ser Ser Lys Val Leu Glu Leu
            180                 185                 190

Cys Gly Asp Leu Pro Ile Phe Trp Leu Lys Pro Thr Tyr Pro Lys Glu
        195                 200                 205

Ile Gln Arg Glu Arg Arg Glu Val Val Arg Lys Ile Val Pro Thr Thr
    210                 215                 220

Thr Lys Arg Pro His Ser Gly Pro Arg Ser Asn Pro Gly Ala Gly Arg
225                 230                 235                 240

Leu Asn Asn Glu Thr Arg Pro Ser Val Gln Glu Asp Ser Gln Ala Phe
                245                 250                 255

Asn Pro Asp Asn Pro Tyr His Gln Gln Glu Gly Glu Ser Met Thr Phe
            260                 265                 270

Asp Pro Arg Leu Asp His Glu Gly Ile Cys Cys Ile Glu Cys Arg Arg
        275                 280                 285

Ser Tyr Thr His Cys Gln Lys Ile Cys Glu Pro Leu Gly Gly Tyr Tyr
    290                 295                 300

Pro Trp Pro Tyr Asn Tyr Gln Gly Cys Arg Ser Ala Cys Arg Val Ile
305                 310                 315                 320

Met Pro Cys Ser Trp Trp Val Ala Arg Ile Leu Gly Met Val
                325                 330

<210> 3
<211> 20
<212> DNA
<213> Artificial

<220>
<223> An artificially synthesized primer sequence

<400> 3
cttaagccca tgtatccaaa 20

<210> 4
<211> 20
<212> DNA
<213> Artificial

<220>
<223> An artificially synthesized primer sequence

<400> 4
cttctagaca cttggtgacc 20

<210> 5
<211> 19
<212> DNA
<213> Artificial

<220>
<223> An artificially synthesized primer sequence

<400>  5
ttcaacggca cagtcaagg                                                    19

<210>  6
<211>  19
<212>  DNA
<213>  Artificial

<220>
<223>  An artificially synthesized primer sequence

<400>  6
gagtactggt gtcaggtac                                                    19

<210>  7
<211>  22
<212>  DNA
<213>  Artificial

<220>
<223>  An artificially synthesized primer sequence

<400>  7
gaaggctcgt attcctgagg tg                                                22

<210>  8
<211>  22
<212>  DNA
<213>  Artificial

<220>
<223>  An artificially synthesized primer sequence

<400> 8
tggcatgatc ttgccttcca gt 22

<210> 9
<211> 26
<212> DNA
<213> Artificial

<220>
<223> An artificially synthesized probe sequence

<400> 9
cgtgaccaaa cagagcatct cctcca 26

<210> 10
<211> 21
<212> RNA
<213> Artificial

<220>
<223> An artificially synthesized siRNA sequence

<400> 10
aaccuccugg caguagaugu a 21

<210> 11
<211> 19
<212> RNA
<213> Artificial

<220>
<223> An artificially synthesized siRNA sequence

<400> 11
cuuacgcuga guacuucga 19
1

## Description

### Technical Field

The present invention relates to therapeutic agents for angiogenesis-related diseases comprising chondromodulin-I as an active ingredient, and methods of screening for therapeutic agents for angiogenesis-related diseases using chondromodulin-I expression as an indicator.

### Background Art

The balance between angiogenic and anti-angiogenic factors is important for normal development and homeostasis of tissues and organs. Heart is a vascular-rich organ and produces many angiogenic factors, while cardiac valves are avascular and oxygen is provided by diffusion from the blood stream (see Non-Patent Document 1). Under pathological conditions such as atherosclerosis, rheumatic valvular heart disease, and infective endocarditis, cardiac valves express angiogenic factors resulting in neovascularization (see Non-Patent Documents 2 and 3). It is unknown that anti-angiogenic factors (angiogenesis-inhibiting factors) are involved in the maintenance of avascularity in cardiac valves.

Cartilage is a typical avascular tissue having characteristics similar to those of cardiac valve tissues. Similar to chondrocytes in cartilage, the mesenchymal cells of cardiac valve tissue, known as valvular interstitial cells (VICs), are sparsely distributed on an incomplete basal lamina, and have direct and extensive contacts with collagen fibers, elastin microfibrils, and proteoglycans of the extracellular matrix underneath the endothelial cell layer (see Non-Patent Documents 4 to 6). Growth factors such as BMP2 (see Non-Patent Document 7) and TGFβ2 (see Non-Patent Document 8), as well as transcription factors essential for cartilage formation during endochondral ossification, such as Sox9 (see Non-Patent Document 9), NATc (see Non-Patent Document 10), Runx2 (also known as Cbfa1) (see Non-Patent Document 11), and MSX2 (see Non-Patent Document 12), are similarly expressed in cardiac valves. It is reported that in conjunction with Sox5 and Sox6, Sox9 can induce even in non-chondrogenic cells expression of genes that are specific to chondromodulin-I (Chm-I)-containing cartilage (see Non-Patent Document 13), and that they are essential to cardiac valve development (see Non-Patent Document 7).

However, a lot is unknown about the action of anti-angiogenic factors in cardiac valves, and the mechanism of how angiogenesis-induced diseases such as valvular heart disease develop has not been elucidated.

The following Patent Documents and Non-Patent Documents are reported as relevant literature of the present invention.
[Patent Document 1] Japanese Patent No. 3585180
[Patent Document 2] Japanese Patent Application Kokai Publication No. (JP-A) H09-299088 (unexamined, published Japanese patent application)
[Patent Document 3] JP-A (Kokai) 2004-123722
[Patent Document 4] JP-A (Kokai) H05-178896
[Non-Patent Document 1] Hammon, J. W., Jr., O'Sullivan, M. J., Oury, J. & Fosburg, R. G. Allograft cardiac valves. A view through the scanning electron microscope. J. Thorac. Cardiovasc. Surg. 68, 352-60 (1974).
[Non-Patent Document 2] Soini, Y., Salo, T. & Satta, J. Angiogenesis is involved in the pathogenesis of nonrheumatic aortic valve stenosis. Hum. Pathol. 34, 756-63 (2003).
[Non-Patent Document 3] Yamauchi, R. et al. Upregulation of SR-PSOX/CXCL16 and recruitment of CD8+ T cells in cardiac valves during inflammatory valvular heart disease. Arterioscler. Thromb. Vasc. Biol. 24, 282-7 (2004).
[Non-Patent Document 4] Filip, D. A., Radu, A. & Simionescu, M. Interstitial cells of the heart valves possess characteristics similar to smooth muscle cells. Circ. Res. 59, 310-20 (1986).
[Non-Patent Document 5] Lester W, R. A., Granton B, et al. Porcine mitral valve interstitial cells in culture. Lab. Invest. 710-719 (1988).
[Non-Patent Document 6] Gotlieb, A. I., Rosenthal, A. & Kazemian, P. Fibroblast growth factor 2 regulation of mitral valve interstitial cell repair in vitro. J. Thorac. Cardiovasc. Surg. 124, 591-7 (2002).
[Non-Patent Document 7] Sugi, Y., Yamamura, H., Okagawa, H. & Markwald, R. R. Bone morphogenetic protein-2 can mediate myocardial regulation of atrioventricular cushion mesenchymal cell formation in mice. Dev. Biol. 269, 505-18 (2004).
[Non-Patent Document 8] Camenisch, T. D. et al. Temporal and distinct TGFbeta ligand requirements during mouse and avian endocardial cushion morphogenesis. Dev. Biol. 248, 170-81 (2002).
[Non-Patent Document 9] Akiyama, H. et al. Essential role of Sox9 in the pathway that controls formation of cardiac valves and septa. Proc. Natl. Acad. Sci. USA 101, 6502-7 (2004).
[Non-Patent Document 10] Ranger, A. M. et al. The transcription factor NF-ATc is essential for cardiac valve formation. Nature 392, 186-90 (1998).
[Non-Patent Document 11] Rajamannan, N. M. et al. Human aortic valve calcification is associated with an osteoblast phenotype. Circulation 107, 2181-4 (2003)
[Non-Patent Document 12] Chan-Thomas, P. S., Thompson, R. P., Robert, B., Yacoub, M. H. & Barton, P. J. Expression of homeobox genes Msx-1 (Hox-7) and Msx-2 (Hox-8) during cardiac development in the chick. Dev. Dyn. 197, 203-16 (1993).
[Non-Patent Document 13] Ikeda, T. et al. The combination of SOX5, SOX6, and SOX9 (the SOX trio) provides signals sufficient for induction of permanent cartilage. Arthritis Rheum. 50, 3561-73 (2004).
[Non-Patent Document 14] Hiraki, Y. et al. Molecular cloning of a new class of cartilage-specific matrix, chondromodulin-I, which stimulates growth of cultured chondrocytes. Biochem. Biophys. Res. Commun. 175, 971-977 (1991).
[Non-Patent Document 15] Funaki, H. et al. Expression and localization of angiogenic inhibitory factor, chondromodulin-I, in adult rat eye. Invest. Ophthalmol. Vis. Sci. 42, 1193-200 (2001). [Non-Patent Document 16] Azizan, A., Holaday, N. & Neame, P. J. Post-translational processing of bovine chondromodulin-I. J. Biol. Chem. 276, 23632-8 (2001).
[Non-Patent Document 17] Hiraki, Y. et al. Identification of chondromodulin I as a novel endothelial cell growth inhibitor. Purification and its localization in the avascular zone of epiphyseal cartilage. J. Biol. Chem. 272, 32419-26 (1997).

### Disclosure of the Invention

### [Problems to be Solved by the Invention]

An objective of the present invention is to analyze the action of anti-angiogenic factors in cardiac valves and such to elucidate the mechanism of how angiogenesis-induced diseases develop. Another objective is to provide therapeutic agents for angiogenesis-induced diseases such as valvular heart diseases, and to provide efficient methods of screening for these therapeutic agents. A further objective of the present invention is to provide artificial heart valves with the function of suppressing angiogenesis in valve tissues.

### [Means for Solving the Problems]

The present inventors carried out dedicated research to solve the above-mentioned objectives. Cardiac valves are recognized as avascular tissues, but their avascularity is lost in several valvular heart diseases (VHDs). To elucidate the molecular mechanism of avascularity in valves, the present inventors analyzed chondromodulin-I (ChM-I) expression in cardiac valves, retina, and such. Furthermore, gene knockout technology was used to investigate the induction of VHD by targeting the ChM-I gene which is considered to be involved in avascularity.

Chondromodulin-I (ChM-I) is a glycoprotein of 121 amino acid residues derived from a type II transmembrane precursor of 335 amino acid residues existing mainly in the avascular tissues of eyes and cartilages (see Non-Patent Documents 14 and 15). After translation, the ChM-I precursor is cleaved by a furin protease at the RERR-ELVR site (see Non-Patent Document 16), and the secreted ChM-I is known to accumulate in the space between cartilage matrix regions (see Non-Patent Document 17).

Chondromodulin-I which is an anti-angiogenic factor isolated from cartilage was detected in the left ventricle, outflow tract, and valve primordia at E9.5, but was detected only in the cardiac valves of late-stage embryos and adults. Significant expression of chondromodulin-I was observed in the normal cardiac valves of mice and humans, but chondromodulin-I decreased significantly in both ApoE^{-/-} mice and human VHDs including infective endocarditis, rheumatic heart disease, and atherosclerosis.

VEGF-A expression, angiogenesis, and calcification were particularly observed in places where chondromodulin-I was down-regulated. The culture supernatant obtained from cultured valvular interstitial cells strongly inhibited tube formation and mobilization of endothelial cells, which were partially inhibited by siRNAs of chondromodulin-I. Furthermore, enhancement of VEGF-A expression, angiogenesis, and change in the valve thickness of cardiac valves occurred when the chondromodulin-I gene was targeted, and echocardiography showed that thickening of the aortic valve was induced and blood flow was disturbed.

As described above, the present inventors succeeded in showing for the first time evidence that chondromodulin-I plays an important role in maintaining a normal valvular function by preventing angiogenesis, thickening, and calcification leading to VHD.

More specifically, inhibition of the expression or function of chondromodulin-I causes abnormalities in cardiac valves and such, and this seems to induce angiogenesis-related diseases such as valvular heart disease as a result. Therefore, the chondromodulin-I protein itself and substances that activate the expression or function of this protein are expected to exhibit effective therapeutic effects on angiogenesis-related diseases such as valvular heart disease.

Furthermore, the present inventors discovered that in patients with angiogenesis-related disease such as valvular heart disease, the expression of chondromodulin-I in cardiac valves was decreased. More specifically, it is possible to screen for therapeutic agents (candidate compounds) for angiogenesis-related diseases by using the expression level or activity of chondromodulin-I as an indicator.

Various findings obtained by the present invention provide new insights into the mechanism of avascularity maintenance in cardiac valves and pathogenesis of angiogenesis-induced diseases. Understanding such mechanism is very important for developing new therapeutic means for valvular heart disease and such, and the present invention makes a great contribution also from an academic standpoint.

Meanwhile, the hybrid-type artificial heart valves produced so far by adding bone marrow cells or such to porcine tissue valves or biodegradable high-molecular compounds (for example, polylactic acid) failed to maintain their function for a long time, and were destroyed as a result of infiltration of new blood vessels into valvular interstitial tissues, and accompanying infiltration of inflammatory cells or activation of matrix metalloprotease (MMP). The artificial heart valves provided by the present invention, however, suppress angiogenesis in valvular tissues, and may contribute to maintain the function for a longer time than conventional artificial heart valves.

Specifically, the present invention relates to therapeutic agents for angiogenesis-induced diseases such as valvular heart disease, and effective methods of screening for such therapeutic agents, and more specifically provides:
[1] an angiogenesis-suppressing agent comprising as an active ingredient any one of:
   (a) a chondromodulin-I protein;
   (b) a protein functionally equivalent to a chondromodulin-I protein, which comprises an amino acid sequence with one or more amino acid deletions, substitutions, or additions in the amino acid sequence of the chondromodulin-I protein; and
   (c) a DNA encoding the protein of (a) or (b);
[2] the angiogenesis-suppressing agent of [1], which has an effect of suppressing angiogenesis in cardiac valves;
[3] the angiogenesis-suppressing agent of [1], which has an effect of suppressing angiogenesis in retina;
[4] a therapeutic agent for angiogenesis-related disease comprising as an active ingredient any one of:
   (a) a chondromodulin-I protein;
   (b) a protein functionally equivalent to a chondromodulin-I protein, which comprises an amino acid sequence with one or more amino acid deletions, substitutions, or additions in the amino acid sequence of the chondromodulin-I protein; and
   (c) a DNA encoding the protein of (a) or (b);
[5] a therapeutic agent for angiogenesis-related disease comprising as an active ingredient a chondromodulin-I protein expression-activating substance or a chondromodulin-I protein function-activating substance;
[6] the therapeutic agent for angiogenesis-related disease of [4] or [5], wherein the angiogenesis-related disease is a disease caused by angiogenesis in cardiac valves;
[7] the therapeutic agent for angiogenesis-related disease of [4] or [5], wherein the angiogenesis-related disease is a disease caused by angiogenesis in retina;
[8] the therapeutic agent for angiogenesis-related disease of [4] or [5], wherein the angiogenesis-related disease is a disease selected from the group consisting of valvular heart disease, infective endocarditis, rheumatic heart disease, atherosclerosis, and retinosis;
[9] the pharmaceutical agent of any one of [1] to [8], wherein the chondromodulin-I protein is a protein comprising the amino acid sequence of SEQ ID NO: 2;
[10] a gene knockout non-human animal, wherein expression of a chondromodulin-I gene is artificially suppressed;
[11] the gene knockout non-human animal of [10], which has an abnormality in its cardiac valve;
[12] the gene knockout non-human animal of [10] or [11], which is used to screen for a therapeutic agent for angiogenesis-related disease;
[13] a method of screening for a therapeutic agent for angiogenesis-related disease, which comprises selecting a compound that activates expression or function of a chondromodulin-I protein;
[14] a method of screening for a therapeutic agent for angiogenesis-related disease, which comprises the steps of:
   (a) contacting a test compound with cells expressing a chondromodulin-I protein;
   (b) measuring expression level of the chondromodulin-I protein in said cells; and
   (c) selecting a compound that increases the expression level compared with that measured in the absence of the test compound;
[15] a method of screening for a therapeutic agent for angiogenesis-related disease, which comprises the steps of:
   (a) contacting a test compound with cells or an extract solution of cells comprising a DNA structured such that a reporter gene is operably linked to the transcriptional regulatory region of a chondromodulin-I gene;
   (b) measuring expression level of said reporter gene; and
   (c) selecting a compound that increases the expression level compared with that measured in the absence of the test compound;
[16] a method of screening for a therapeutic agent for angiogenesis-related disease, which comprises the steps of:
   (a) contacting a test compound with a chondromodulin-I protein, or cells or an extract solution of cells expressing said protein;
   (b) measuring activity of said protein; and
   (c) selecting a compound that increases the protein activity compared with that measured in the absence of the test compound;
[17] a method of screening for a therapeutic agent for angiogenesis-related disease, which comprises the steps of:
   (a) administering a test compound to the gene knockout non-human animal of any one of [10] to [12];
   (b) measuring the expression level or activity of chondromodulin-I protein in said gene knockout non-human animal; and
   (c) selecting a compound that increases the expression level or activity of chondromodulin-I protein compared with when the test compound is not administered;
[18] the screening method of [17], wherein the expression level or activity of said protein in the cardiac valve or eye of said non-human animal is measured;
[19] a method of screening for a therapeutic agent for angiogenesis-related disease, which comprises the steps of:
   (a) administering a test compound to the gene knockout non-human animal of any one of [10] to [12];
   (b) observing the cardiac valve or retina in said gene knockout non-human animal; and
   (c) selecting a compound that normalizes the cardiac valve or retina compared with when the test compound is not administered;
[20] the screening method of any one of [13] to [19], wherein the angiogenesis-related disease is valvular heart disease, infective endocarditis, rheumatic heart disease, atherosclerosis, or retinosis;
[21] an artificial heart valve comprising as major components:
   (a) cells expressing a chondromodulin-I gene; and
   (b) a decellularized valve or biodegradable high-molecular compound; and
[22] an artificial heart valve produced by the steps of:
   (a) culturing and growing cells that express a chondromodulin-I gene;
   (b) seeding the cells of (a) onto the surface of a decellularized valve or biodegradable high-molecular compound; and
   (c) culturing until the cells expressing the chondromodulin-I gene fill the pore spaces of the decellularized valve or biodegradable high-molecular compound of (b).

The present invention further relates to methods for producing angiogenesis-suppressing agents and therapeutic agents for angiogenesis-related diseases, which comprise the step of mixing any of the following (a) to (c) with pharmaceutically acceptable carriers or vehicles:
(a) a chondromodulin-I protein (for example, the human chondromodulin-I protein as shown in the amino acid sequence of SEQ ID NO: 2);
(b) a protein functionally equivalent to a chondromodulin-I protein, which comprises an amino acid sequence with one or more amino acid deletions, substitutions, or additions in the amino acid sequence of the chondromodulin-I protein (for example, the human chondromodulin-I protein amino acid sequence of SEQ ID NO: 2); and
(c) a DNA encoding the protein of (a) or (b) above.

Furthermore, the present invention relates to methods for preventing or treating angiogenesis-related diseases, which comprise the steps of administering to an individual any of (a) to (c) mentioned above. The present invention also relates to methods for treating angiogenesis-related diseases comprising the steps of using the artificial heart valve of [21] or [22].

Alternatively, the present invention provides use of any of the substances of (a) to (c) mentioned above in the production of angiogenesis-suppressing agents and therapeutic agents for angiogenesis-related diseases.

### Brief Description of the Drawings

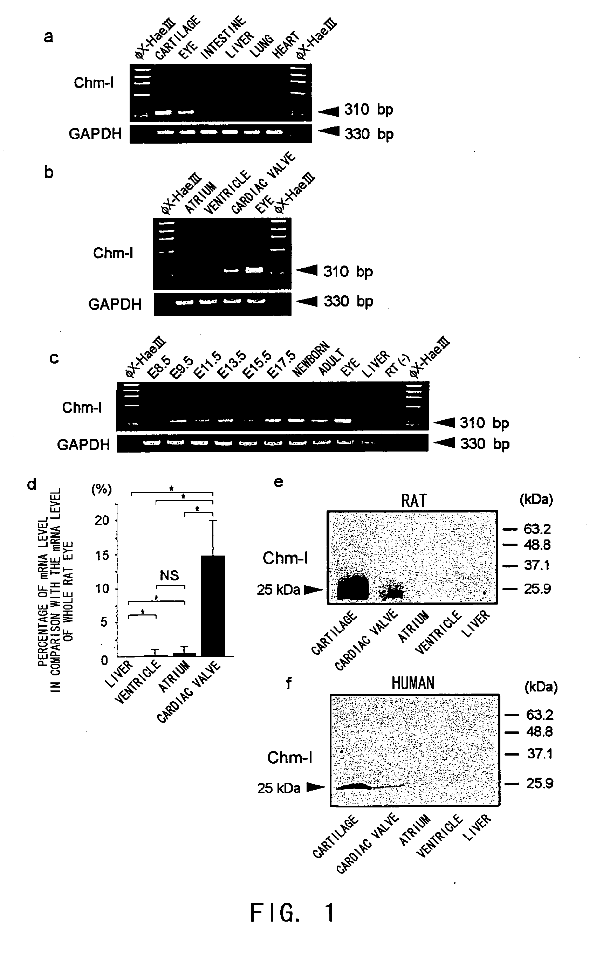

Figure 1 shows photographs and a diagram showing the temporal and spatial expressions of ChM-I in rodent and human hearts.
   (a) A photograph showing the RT-PCR result of chm-I using total RNAs from various rat tissues. The cartilage, eye, and heart showed positive signals (310 bp).
   (b) A photograph showing the spatial expression of chm-I in rat heart. Positive signal was observed in the cardiac valve but not in the atrium and ventricle. Adult rat eye was used as a positive control.
   (c) A photograph showing the temporal expression of chm-I in rat heart. RT PCR was performed on embryonic and adult hearts. Positive signals were observed in embryonic hearts from E9.5 and later. Adult rat eye and liver were used as positive and negative controls, respectively.
   (d) A graph showing quantitative PCR result of chm-I in adult rat heart. The expression level of chm-I in the heart valve was 30.3 times higher than in the atrium and 72.1 times higher than in the ventricle. *P<0.01.
   (e, f) Photographs showing the result of Western blot analysis of rat heart (e) and human heart (f) using an anti-ChM-I antibody. Cartilage and liver were used as positive and negative controls, respectively.

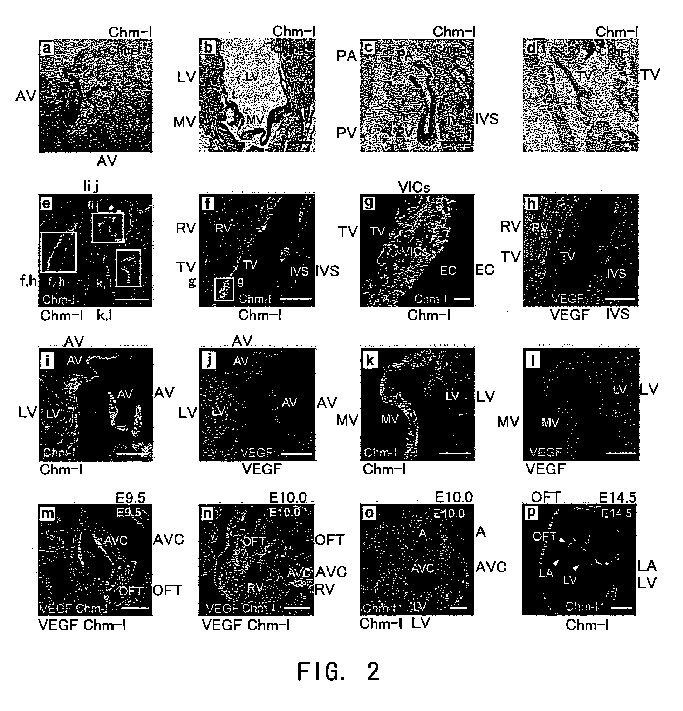

Figure 2 shows photographs of the results of immunohistochemistry and immunofluorescent staining of the ChM-I protein in developing and adult mouse hearts.
   (a to d) ChM-I was expressed in all four valves in the adult. Positive signals are shown in brown. (a, b) Short-axis views. (c, d) Long-axis views. AV, aortic valve; LV, left ventricle; MV mitral valve; PV, pulmonary valve; PA, pulmonary artery; IVS, interventricular septum.
   (e to p) Immunofluorescent staining of ChM-I and VEGF-A is shown in the serial sections of adult (e to i) and developmental-stage (m to p) mouse hearts. A, atrium; LA, left atrium; TV, tricuspid valve; RV, right ventricle; VIC, ventricular interstitial cells; EC, endothelial cells; AVC, atrioventricular cushion; OFT, outflow tract.

   The bars represent 1 mm (e and p), 200 µm (a, b, d, f, and h to n), 100 µm (c and o), and 20 µm (g).

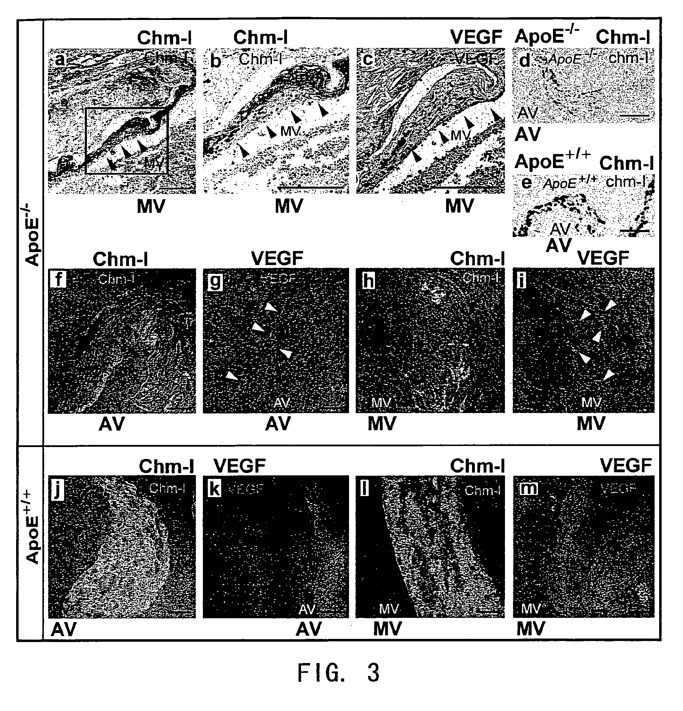

Figure 3 shows photographs of the results of immunohistochemistry, immunofluorescent staining, and *in situ* hybridization of ChM-I and VEGF-A in sclerotic lesions of aged ApoE^{-/-} mouse hearts.
   (a) Immunohistochemistry of ChM-I and VEGF-A in a sclerotic lesion (arrowheads) of the mitral valve (MV) of an ApoE^{-/-} mouse heart. The boxed area is enlarged in (b).
   (c) Immunostaining of the serial section (b) using a VEGF-A antibody. (d, e) *In situ* hybridization of ChM-I in ApoE^{-/-} and age-matched ApoE^{+/+} mouse aortic valves (AV).
   (f to m) Immunofluorescent staining of ChM-I and VEGF-A in ApoE^{-/-} (f to i) and age-matched ApoE^{+/+} mice (j to m). It should be noted that in ApoE^{-/-} mice VEGF-A was markedly up-regulated in the sclerotic valve area where ChM-I was down-regulated.

   The bars represent 100 µm (a to c) and 20 µm (d to m).

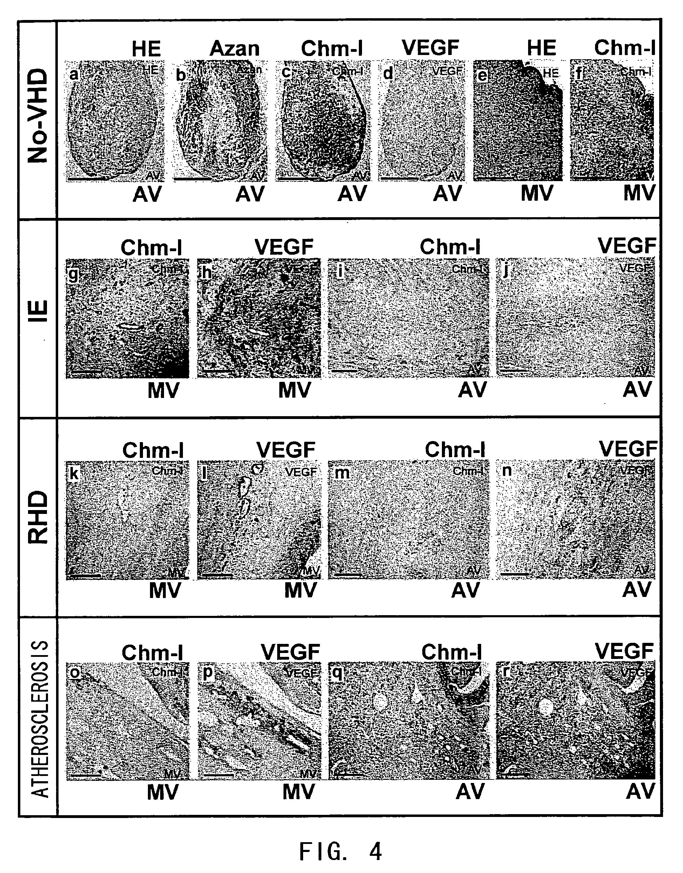

Figure 4 shows photographs of the results of histology and immunohistochemistry for the cardiac valves in human autopsy samples and surgical samples.
   (a to f) No-VHD (no valvular heart disease) autopsy samples. HE, hematoxylin-eosin staining; Azan, Azan staining. It should be noted that ChM-I was strongly expressed but VEGF-A was not expressed.
   (g to r) Representative micrographs of immunohistochemistry of the surgical samples from various valvular heart diseases. Significant angiogenesis was observed in infective endocarditis (IE, g to j), rheumatic heart disease (RHD, k to n), and atherosclerosis-valvular heart disease (o to r). ChM-I decreased significantly in large regions where new blood vessel formation was observed and VEGF-A was strongly expressed.

   AV, aortic valve; MV, mitral valve. The bar represents 200 µm.

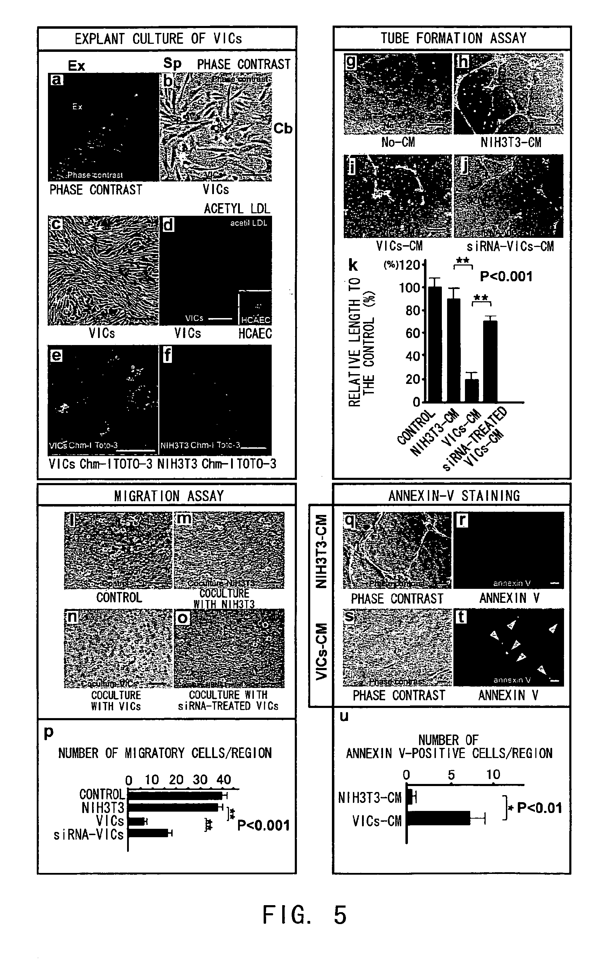

Figure 5 shows photographs and diagrams of ChM-I expression in ventricular interstitial cells (VICs) and its effect *on in vitro* tube formation, migration, and apoptosis in human coronary artery endothelial cells (HCAECs).
   (a) A phase-contrast micrograph of the monolayer culture of rat VICs three days after explant culture.
   (b) Seven days after explant culture. VICs showed a cobblestone-like (Cb) or spindle-like (Sp) appearance.
   (c) Fourteen days after explant culture, VICs showed a fibroblast-like appearance at confluent density.
   (d) VICs shown in Fig. 5b were negative for acetyl-LDL-DiI staining. HCAECs are shown as a positive control (inset).
   (e, f) Immunofluorescent staining of ChM-I in VICs (c) and NIH3T3 cells (f). Nuclei of both cells were stained with TOTO-3. ChM-I was stained in a granular pattern in the cytoplasm.
   (g to k) VIC-conditioned medium *inhibited in vitro* endothelial cell tube formation on Matrigel. (g) A representative micrograph of tube formation for HCAECs on Matrigel. CM, conditioned medium. (h) NIH3T3-CM did not affect the tube formation in HCAECs. (i) VIC-CM significantly suppressed the tube formation in HCAECs after six hours. (j) VICs were treated with chm-I-specific siRNA for three days. siRNA-treated VIC-CM partially reduced the tube formation-suppressing ability in HCAECs. (k) Data from quantitatively analyzing (g) to (j) using the Scion Image software. The tube lengths of the cells were measured in five different 0.25 mm² regions from three experiments, and the lengths were shown in mm/mm² as described in the Methods.
   (1 to p) VICs inhibited HCAEC chemotaxis *in vitro.* HCAECs were applied to the upper chamber and cocultured with VICs, and dropped to the lower chamber (m) of the Boyden chamber. After incubation (37°C, three hours), migration of HCAECs to the underside of the membrane was inhibited compared with when cultured without any cells (1) or when cocultured with NIH3T3 cells. (o) chm-I-specific siRNAs decreased the VIC-CM-induced suppression of HCAEC chemotaxis. (p) For each experiment, five high-powered fields were randomly selected from triplicate experiments and the results of determining the number of cells are shown. The graph shows the number of migratory cells.
   (q to u) Induction of apoptosis by VIC-CM. HCAECs cultured with NIH3T3-CM (q, r) or VICs-CM (s, t) were treated with FITC-conjugated Annexin V. The present inventors detected Annexin V-positive fluorescent cells when HCAECs were cultured with VIC-CM for six hours. (u) Five visual fields were randomly selected from triplicate experiments and apoptotic cells were quantified using the number of Annexin V-FITC-positive cells.

   The bars represent 50 µm (e, f), 100 µm (a to d, 1 to o, and q to t), and 200 µm (g to j). *, P < 0.05; **, P < 0.01.

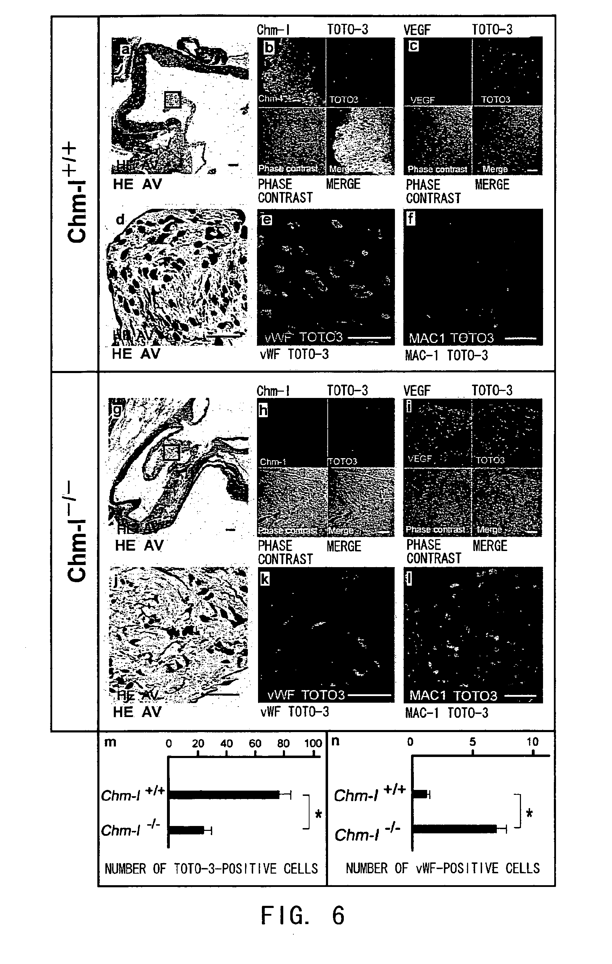

Figure 6 shows photographs and diagrams of abnormal angiogenesis, inflammatory cell infiltration, and mineralization in the cardiac valves of aged chm-I^{-/-} mice.
   (a) Thickened valves of aged chm-I^{-/-} mice were shown by HE staining at low magnification.
   (b) The chm-I^{-/-} mice were confirmed to be negative for ChM-I immunostaining.
   (c) A chm-I^{-/-} mouse valve was stained with VEGF-A and strong positive signals in the aortic valve were observed.
   (d) Capillary-like structures were found in aged chm-I^{-/-} mice at high magnification by HE staining and by positive vWF staining of the serial section (e). The other serial section showed positive staining for MAC-1 immunofluorescence (1). Compared with chm-I^{-/-} mice, the age-matched chm-I^{+/+} mice used as a control were positive for ChM-I (h), and negative for VEGF-A-positive cells (i), capillary-like structures (j), vWF (k), and MAC-1 (1).
   (m, n) For at least the valve that is visible with immunofluorescence, the numbers of TOTO-3-positive cells and vWF-positive cells in three lobules of the aortic valve were counted for every 20 µm-section stained. Graphs were produced for chm-I^{-/-} (n = 7) and chm-I^{+/+} (n = 5).

   The bars represent 50 µm (a, f, g, and 1) and 20 µm (others). *, P < 0.01.

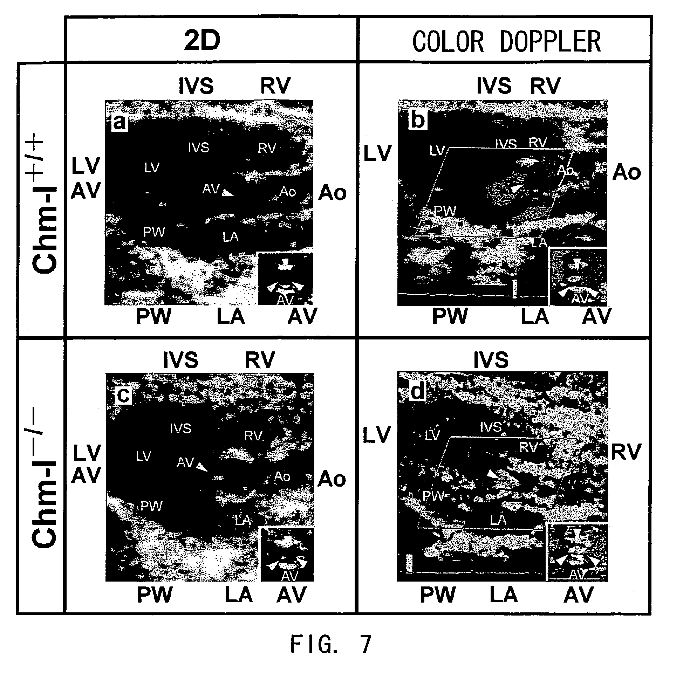

Figure 7 shows photographs on the results of echocardiography of chm-I^{-/-} mouse hearts.
   (a) Two-dimensional (2D)-mode echocardiography showed an abnormal bright echogenic mass in the aortic valve, but this was not detected in chm-I^{+/+} mouse heart (c).
   (b) Color Doppler study showed a mosaic turbulent flow that was not detected in the aortic valve region of the chm-I^{+/+} mouse heart.
   (d) The insets show the AV-level short-axis view of each long-axis figure.

   AV, aortic valve; RV, right ventricle; LV, left ventricle; LA, left ventricle; LA, left atrium; Ao, aorta; IVS, interventricular septum; PW, posterior wall.

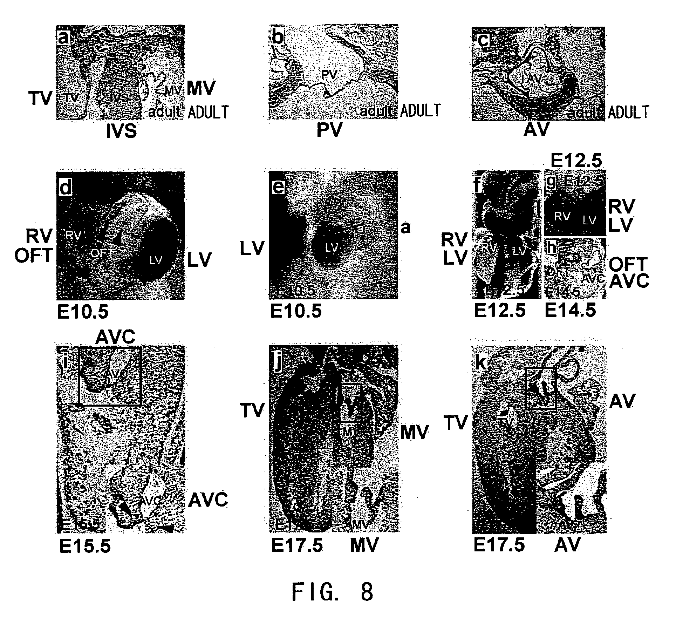

Figure 8 shows photographs for the results of *in situ* hybridization for chm-I in mouse hearts.
   (a, c) Presence of the chm-I mRNA was confirmed in four valves of adult mouse hearts.
   (d, e) Whole mount *in situ* hybridization (WISH method) of E10.5 mouse embryonic heart showed positive signals in the outflow tract and left ventricle, and weak signals in the outer curvature of the right ventricle and atrium (f and g). The same signal transduction pattern was observed for E12.5. In these embryonic stages, the heart was the organ showing the strongest positive signal against the chm-I-specific probe when observed under a stereoscopic microscope (f). Other tissues were removed and the heart was isolated (g).
   (h to k) During development, expression of the chm-I mRNA became localized in the cardiac valve primordia and epicardium. (j, k) In late stage embryogenesis, signals could not be observed in the cardiomyocytes of LV, and thin valves caused by apoptosis in the atrioventricular cushions and outflow tract showed brown positive signals for the chm-I mRNA.

   TV, tricuspid valve; MV, mitral valve; PV, pulmonary valve; AV, aortic valve; RV, right ventricle; LV, left ventricle; A, atrium; OFT, outflow tract; AVC, atrioventricular cushion.

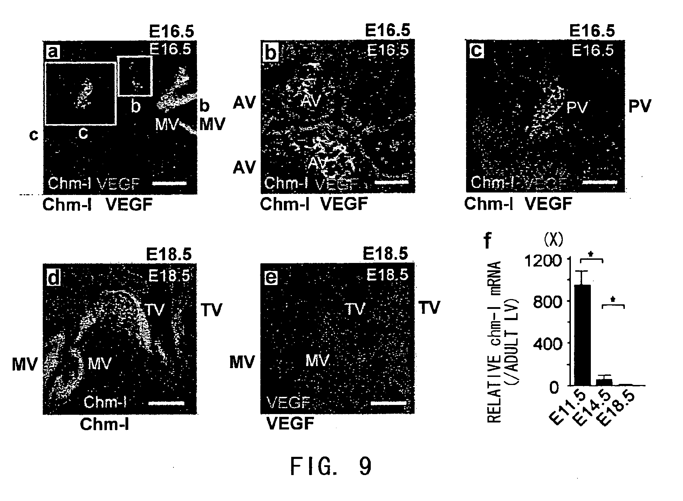

Figure 9 shows photographs and a diagram for the results of immunofluorescent staining of chm-I in mid-stage and late-stage embryonic mouse hearts.
   (a to c) E16.5 embryonic mouse hearts were immunostained with anti-ChM-I and anti-VEGF-A. The cardiac valve primordia showed positive signals for ChM-I. AV and PV regions were enlarged in (b) and (c), respectively. In contrast to ChM-I expression, VEGF-A expression was observed in cardiomyocytes and endothelial cells.
   (d, e) E18.5 embryonic mouse heart immediately before birth. Cardiac valves were nearly formed, and were ChM-I-positive and VEGF-A-negative.
   (f) QT-PCR was performed for chm-I with heart extracts of E11.5, E14.5, and E18.5 embryos. The chm-I expression recognized in the left ventricle of early developmental stage embryos rapidly decreased by the mid-stage of development.

   The bars represent 200 µm (a, d, and e) and 50 µm (b and c).

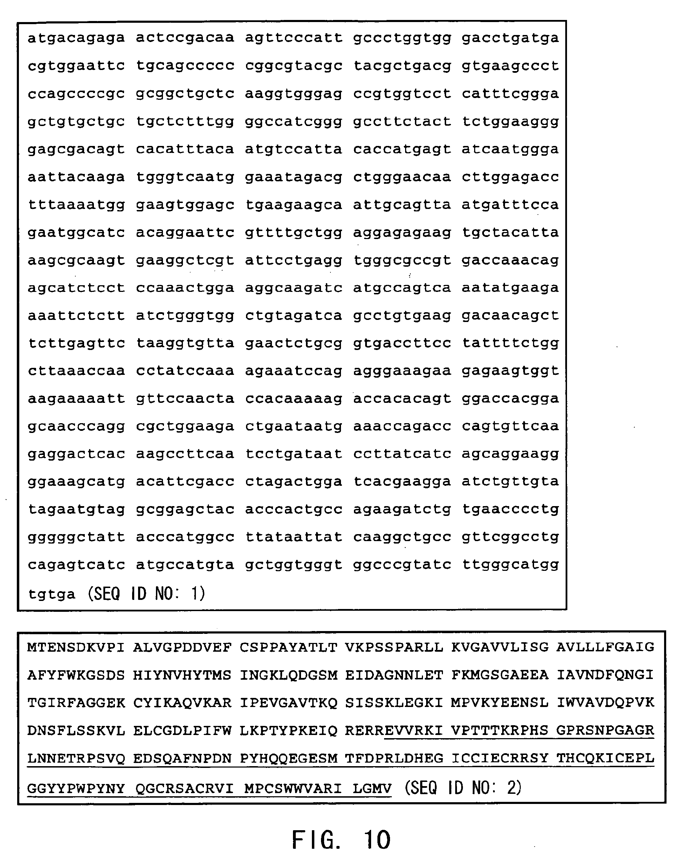

Figure 10 shows the nucleotide sequence of a human chondromodulin-I gene, and the amino acid sequence of a human chondromodulin-I protein. The amino acid sequence is that of a human chondromodulin-I precursor protein determined from its cDNA nucleotide sequence , and the C-terminal portion, a 120-amino acid residue portion (underlined), is the amino acid sequence of a (mature) human chondromodulin-I protein.

### Best Mode for Carrying Out the Invention

Herein below, the present invention will be described in detail.

The present inventors showed that inhibition of the expression or function of chondromodulin-I causes abnormalities in cardiac valves and such, and as a result, angiogenesis-related diseases of cardiac valves and such arise. Therefore, the chondromodulin-I protein itself or substances that activate the expression or function of this protein are expected to exhibit effective therapeutic effects against angiogenesis-related diseases such as valvular heart disease.

First, the present inventors provide therapeutic agents for angiogenesis-related diseases comprising a chondromodulin-I protein as an active ingredient.

The chondromodulin-I (ChM-I) protein of the present invention is preferably a human chondromodulin-I protein, but the biological species from which it is derived is not particularly limited, and proteins equivalent to chondromodulin-I found in non-human animals (homologs, orthologs; or such of human chondromodulin-I) are also included in the "chondromodulin-I protein" of the present invention. The present invention can be carried out, for example, if the organism has cardiac tissues, vascular tissues, and such, and has a protein equivalent to human chondromodulin-I.

For example, the amino acid sequence of human chondromodulin-I protein is shown in SEQ ID NO: 2, and the nucleotide sequence of a DNA encoding this amino acid sequence (chondromodulin-I gene) is shown in SEQ ID NO: 1.

Examples of organisms other than human (Accession No. AB006000) that have a protein corresponding to chondromodulin-I include mouse (Accession No. U43509), rat, rabbit, cattle (Accession No. M65081), chicken (Accession No. AF027380.1), zebrafish, Xenopus (Accession No. BC043890), and medaka.

For example, proteins that are highly homologous to the sequence shown in the sequence listing of the present application (usually 70% or more, preferably 80% or more, more preferably 90% or more, and most preferably 95% or more) and have a function of chondromodulin-I (for example, the activity to inhibit angiogenesis) may be included in the chondromodulin-I of the present invention, even if they are proteins not mentioned above. The above-mentioned proteins are, for example, a protein comprising an amino acid sequence with one or more amino acid additions, deletions, substitutions, or insertions in the amino acid sequence of SEQ ID NO: 2, and the number of amino acids that are modified is usually 30 amino acids or less, preferably ten amino acids or less, more preferably five amino acids or less, and most preferably three amino acids or less.

"Chondromodulin-I gene" of the present invention includes, for example, endogenous genes from other organisms that correspond to a DNA comprising the nucleotide sequence of SEQ ID NO: 1 (for example, homologs and such of the human chondromodulin-I gene).

Endogenous DNAs from other organisms corresponding to the DNA comprising the nucleotide sequence of SEQ ID NO: 1 are generally highly homologous to the DNA of SEQ ID NO: 1. Highly homologous refers to a homology of 50% or more, preferably 70% or more, more preferably 80% or more, and even more preferably 90% or more (for example, 95% or more, or 96%, 97%, 98%, or 99% or more). This homology can be determined using the mBLAST algorithm (Altschul et al., Proc. Natl. Acad. Sci. USA 87, 2264-8 (1990); Karlin and Altschul, Proc. Natl. Acad. Sci. USA 90, 5873-7 (1993)). Furthermore, when these DNAs are isolated from living bodies, they are expected to hybridize with the DNA of SEQ ID NO: 1 under stringent conditions. Herein, examples of "stringent conditions" include conditions such as "2x SSC, 0.1% SDS, 50°C", "2x SSC, 0.1% SDS, 42°C", and "1x SSC, 0.1% SDS, 37°C", and more stringent conditions such as "2x SSC, 0.1% SDS, 65°C", "0.5x SSC, 0.1% SDS, 42°C", and "0.2x SSC, 0.1% SDS, 65°C". Those skilled in the art can suitably obtain in other organisms endogenous genes corresponding to the chondromodulin-I gene based on the nucleotide sequence of the chondromodulin-I gene. In this description, proteins (genes) corresponding to chondromodulin-I proteins (genes) in non-human animals, and proteins (genes) functionally equivalent to the above-described chondromodulin-I may be simply referred to as "chondromodulin-I proteins (genes)" or "ChM-I".

"Chondromodulin-I protein" of the present invention may be a naturally-occurring protein, or can be prepared as a recombinant protein using gene recombination techniques. Naturally-occurring proteins can be prepared, for example, from an extract solution of cells (tissues) that are considered to be expressing a chondromodulin-I protein, by an affinity chromatography method using antibodies against the chondromodulin-I protein. Meanwhile, recombinant proteins can be prepared by culturing cells that have been transformed with a chondromodulin-I protein-encoding DNA. "Chondromodulin-I protein" of the present invention is suitably used, for example, in the screening methods described later, as a control protein or such.

In the present invention, "expression" includes "transcription" from genes, "translation" into polypeptides, and "suppression of degradation" of proteins. "Expression of chondromodulin-I protein" means that transcription and translation of a gene encoding the chondromodulin-I protein take place, or that the chondromodulin-I protein is produced by the transcription and translation. Furthermore, "chondromodulin-I protein function" refers to, for example, function to inhibit angiogenesis, function to promote DNA synthesis in a costochondral cell culture system (Y. Hiraki, et al., Biochem. Biophys. Res. Commun. 175, 971-977 (1991)), function to promote proteoglycan synthesis in a costochondral cell culture system (H. Inoue, et al., Biochem. Biophys. Res. Commun. 241, 395-400 (1997); Y. Hiraki, et al., J. Biol. Chem. 272, 32419-32426 (1997)), function to promote colony formation in a costochondral cell culture system (H. Inoue, et al., Biochem. Biophys. Res. Commun. 241, 395-400 (1997)), function to inhibit DNA synthesis and cell growth in an arterial endothelial cell culture system (Y. Hiraki, et al., J. Biol. Chem. 272, 32419-32426 (1997)), function to inhibit tube formation in an arterial endothelial cell culture system (Y. Hiraki, et al., J. Biol. Chem. 272, 32419-32426 (1997); Y. Hiraki, et al., Eur. J. Biochem. 260, 869-878 (1999)), function to inhibit angiogenesis in the chick embryo chorioallantoic membrane (Y. Hiraki, et al., Eur. J. Biochem. 260, 869-878 (1999)), function to inhibit tumor outgrowth and tumor angiogenesis induced by transplanted human tumors (T. Hayami, et al., FEBS Lett. 458, 436-440 (1999); Y. Oshima, et al., J. Cell Sci. 117, 2731-2744 (2004)), function to inhibit DNA synthesis and tube formation in cultured human retinal vascular endothelial cells (H. Funaki, et al., Invest. Ophthalmol. Vis. Sci. 42, 1193-1200 (2001); Y. Oshima, et al., Invest. Ophthalmol. Vis. Sci. 44, 1814-1823 (2003)), inhibitory function against DNA synthesis, cell migration, and tube formation in cultured human umbilical vein endothelial cells (Y. Oshima, et al., Invest. Ophthalmol. Vis. Sci. 44, 1814-1823 (2003); Y. Oshima, et al., J. Cell Sci. 117, 2731-2744 (2004)), and activity to suppress rheumatoid arthritis: inhibitory function against T-cell immune response and proliferation of cultured synoviocytes (K. Setoguchi, et al., Arthritis Rheumatism 50, 828-839 (2004)).

Each of the functions described above can be evaluated (measured) appropriately by those skilled in the art using conventional techniques. Specifically, they can be performed by referring to the literature and such mentioned for each of the above-described functions.

The angiogenic-suppressing activity of chondromodulin-I proteins can be measured by appropriately evaluating, without limiting thereto, (1) migratory activity of vascular endothelial cells, (2) induction of apoptosis of vascular endothelial cells, and (3) tube formation reaction of vascular endothelial cells, and such.

The present invention provides therapeutic agents for angiogenesis-related diseases comprising as an active ingredient the human chondromodulin-I protein or a functionally equivalent variant of this chondromodulin-I protein (a modified form, homolog of other organisms, or such).

More specifically, a preferred embodiment of the present invention relates to therapeutic agents for angiogenesis-related diseases which comprise the substance of (a) or (b) below as an active ingredient:
(a) a chondromodulin-I protein; or
(b) a protein functionally equivalent to a chondromodulin-I protein, which comprises an amino acid sequence with one or more amino acid deletions, substitutions, or additions in the amino acid sequence of the chondromodulin-I protein (SEQ ID NO: 2).

"Chondromodulin-I protein" which is an ingredient of the pharmaceutical agents of the present invention may be a naturally-occurring protein, or can be prepared as a recombinant protein using gene recombination techniques. Naturally-occurring proteins can be prepared, for example, from an extract solution of cells (tissues) thought to be expressing a chondromodulin-I protein, by an affinity chromatography method using antibodies against the chondromodulin-I protein. Meanwhile, recombinant proteins can be prepared by culturing cells that have been transformed with a chondromodulin-I protein-encoding DNA.

Chondromodulin-I protein-encoding DNA which is a component of the pharmaceutical agents of the present invention is also included in the present invention. The chondromodulin-I protein-encoding DNA of the present invention may be a chromosomal DNA or a cDNA. Chondromodulin-I protein-encoding chromosomal DNA can be obtained, for example, by preparing a chromosomal DNA library from cells and such, and screening this library using probes that hybridize to the chondromodulin-I protein-encoding DNA. The chondromodulin-I protein-encoding cDNA can be obtained by extracting an RNA sample from cells (tissues) that are thought to be expressing a chondromodulin-I protein, and using gene amplification techniques such as RT-PCR with primers that hybridize to the chondromodulin-I protein-encoding DNA.

The chondromodulin-I protein of the present invention and a DNA encoding the protein may be a variant with modified nucleotide sequences or amino acid sequences, so long as it is functionally equivalent to the chondromodulin-I protein. Such variants may be naturally-occurring or artificial. Methods for artificially preparing variants are well known to those skilled in the art. Known examples include the Kunkel method (Kunkel, T. A. et al., Methods Enzymol. 154, 367-382 (1987)), the double primer method (Zoller, M. J. and Smith, M., Methods Enzymol. 154, 329-350 (1987)), cassette mutagenesis (Wells, et al., Gene 34, 315-23 (1985)), and the megaprimer method (Sarkar, G. and Sommer, S. S., Biotechniques 8, 404-407 (1990)).

The pharmaceutical agents of the present invention may comprise as a component, a DNA encoding the protein of (a) or (b) described above, or a vector for expressing the protein. Specifically, the present invention relates to therapeutic agents for angiogenesis-related diseases comprising as an active ingredient:
(c) a DNA encoding the protein of (a) or (b) mentioned above, or a vector that can express the DNAs.

The above-mentioned DNA is preferably operably linked to a promoter for efficient expression. The original chondromodulin-I gene promoter can be used as a promoter for the present invention, but besides this, a variety of known promoters such as a CMV promoter can be used. Furthermore, those skilled in the art can easily produce vectors for expressing the proteins of the present invention using a variety of known expression vectors.

The above-mentioned vectors of the present invention can also be used for gene therapy. Gene therapy refers to administering a vector comprising a DNA encoding a functional protein to patients for therapy or prevention. Vectors that may be used for gene therapy are, for example, adenovirus vector (such as pAdex1cw) and retrovirus vector (for example, pZIPneo), but are not limited thereto. Conventional genetic manipulation such as insertion of a DNA encoding a protein of the present invention into a vector can be performed by ordinary methods. Administration into a living body may be carried out by *ex vivo* methods, *but in vivo* methods are preferred.

Activation of the expression or function of a chondromodulin-I protein of the present invention suppresses angiogenesis, and as a result, is expected to obtain therapeutic effects for angiogenesis-related diseases.

Accordingly, the present invention provides therapeutic agents for angiogenesis-related diseases comprising as an active ingredient a chondromodulin-I protein expression-activating substance or a chondromodulin-I protein function-activating substance.

In the present invention, "angiogenesis-related diseases" refers to angiogenesis-induced diseases, and in particular, they are preferably angiogenesis-induced diseases in cardiac valves or retina. Examples of angiogenesis-related diseases in the present invention include valvular heart disease, infective endocarditis, rheumatic heart disease, atherosclerosis, retinosis, angiogenesis from the surrounding area to the surface of cornea, angiogenesis of cancer, and arthritis such as chronic rheumatoid arthritis.

The "protein expression-activating substance" of the present invention is a substance that significantly activates (increases) protein expression. The above-mentioned "expression-activating" in the present invention includes activation of transcription of a gene encoding the protein and/or activation of translation from the transcriptional product of the gene.

Examples of chondromodulin-I protein expression-activating substances of the present invention include substances that promote transcription (for example, a transcriptional activator) of a chondromodulin-I gene by binding to the transcriptional regulatory domain of the gene (for example, a promoter region).

The expression activity of chondromodulin-I protein in the present invention can be easily measured by those skilled in the art using a known method such as Northern blotting and Western blotting.

The chondromodulin-I protein function-activating substances refers to substances that significantly activate chondromodulin-I protein function. The present inventors showed that the chondromodulin-I protein has, for example, a function of suppressing angiogenesis in cardiac valves. Accordingly, examples of the function-activating substances of the present invention include substances that enhance the angiogenesis-suppressing effect of chondromodulin-I protein in cardiac valves.

The chondromodulin-I protein and substances that activate the expression or function of this protein have the effect of suppressing angiogenesis (for example, angiogenesis in cardiac valves). Accordingly, the present invention provides angiogenesis-suppressing agents (inhibitors) comprising as an active ingredient any of (a) to (c) mentioned above.

The above-mentioned angiogenesis-suppressing agents of the present invention have angiogenesis-suppressing effects preferably in cardiac valves, retina, cornea, articular cartilages, or tumor tissues.

"Therapeutic agents" of the present invention can also be expressed as "pharmaceuticals", "pharmaceutical compositions", "therapeutic pharmaceuticals", or such.

"Treatment" in the present invention includes preventive effects that may suppress the development of a disease in advance, and is not necessarily limited to cases showing complete therapeutic effects; cases showing partial effects, cases showing improvement of symptoms, and such are also included in the meaning of "treatment" in the present invention.

The pharmaceutical agents of the present invention (angiogenesis-suppressing agents, therapeutic agents for angiogenesis-related diseases, and such) can be administered orally or parenterally as pharmaceutical compositions by mixing with physiologically acceptable carriers, excipients, diluents, and such. The dosage form of the oral agent can be granules, powders, tablets, capsules, solutions, emulsions, suspensions, or such. The dosage form of the parenteral agent can be selected from injections, drip infusions, external preparations, suppositories, and such. Injections may be subcutaneous injections, intramuscular injections, intraperitoneal injections, or such. External preparations may be agents for nasal administration, ointments, or such. Techniques to formulate these dosage forms to comprise the agent of the present invention as the principal component are well known.

Tablets for oral administration can be produced, for example, by combining the agent of the present invention with excipients, disintegrating agents, binding agents, lubricants, and such, and press-forming the mixture. Lactose, starch, mannitol, and such are generally used as excipients. Calcium carbonate, calcium carboxymethyl cellulose, and such are generally used as disintegrating agents. Gum Arabic, carboxymethyl cellulose, or polyvinylpyrrolidone is used as a binding agent. Known lubricants include talc and magnesium stearate.

A tablet comprising the agent of the present invention can be coated for masking or for forming enteric-coated preparations according to conventional methods. Ethyl cellulose, polyoxyethyleneglycol, and such can be used as a coating agent.

An injection can be obtained by dissolving the agent of the present invention as the principal component together with an appropriate dispersant, or dissolving or dispersing the agent in a dispersion medium. Depending on the type of dispersion medium selected, the dosage form can be an aqueous solution or an oleaginous solution. To prepare aqueous solutions, distilled water, physiological saline, Ringer solution, or such is used as a dispersion medium. Various vegetable oils, propyleneglycol, and such are used as dispersion media for preparing oleaginous solutions. Preservatives such as paraben can also be added if necessary. Known isotonizing agents such as sodium chloride and glucose can be added to the injections. Soothing agents such as benzalkonium chloride and procaine hydrochloride can be further added.

An agent of the present invention can be formulated for external use by preparing a solid, liquid, or semi-solid composition. The solid and liquid compositions can be made into external preparations by forming the same compositions as those mentioned above. The semi-solid preparations can be prepared by adding a thickening agent to an appropriate solvent when necessary. Water, ethyl alcohol, polyethylene glycol, or such can be used for the solvent. Commonly used thickening agents include bentonite, polyvinyl alcohol, acrylic acid, methacrylic acid, and polyvinylpyrrolidone. Preservatives such as benzalkonium chloride may be added to this composition. An agent of the present invention can also be formulated into a suppository by the combined use of an oily base such as cacao butter, or an aqueous gel base such as cellulose derivatives, as a carrier.

When using an agent of the present invention as an agent for gene therapy, the agent of the present invention may be administered directly by injection, or as a vector incorporating the nucleic acid. Examples of the vector include adenovirus vectors, adeno-associated virus vectors, herpes virus vectors, vaccinia virus vectors, retrovirus vectors, and lentivirus vectors. Efficient administration is possible with the use of these virus vectors.

Alternatively, an agent of the present invention may be introduced into a phospholipid vesicle such as a liposome, and that vesicle can be administered. More specifically, vesicles carrying an agent of the present invention are introduced into given cells by the lipofection method. Cells obtained as a result are systemically administered into veins, arteries, or such. Local administration to cardiac valves, retina, or such is also possible.

A required amount (effective amount) of a pharmaceutical agent of the present invention is administered to animals including humans within a dosage range that is considered to be safe. The dosage of a pharmaceutical agent of the present invention can be determined appropriately by considering the type of dosage form, method of administration, age and body weight of patients, symptoms of patients, and such and ultimately by the decision made by a physician or a veterinarian.

The present invention further provides chondromodulin-I gene knockout non-human animals (in the present description, they may be described as "knockout non-human animal(s)" or simply "animal(s)") in which the expression of a chondromodulin-I gene of the present invention is artificially suppressed.

The gene knockout non-human animals of the present invention can be used, for example, to screen for pharmaceutical agents to treat angiogenesis-related diseases. They are also very useful as pathological model animals in research for elucidating the mechanisms of these diseases.

The knockout animals of the present invention also include the so-called "knockdown animals" whose gene expression is suppressed by the action of antisense RNA or siRNA.

Examples of conditions where "chondromodulin-I gene expression is artificially suppressed" in the present invention include (1) conditions where the expression of chondromodulin-I gene is suppressed by the presence of genetic mutations such as nucleotide insertions, deletions, or substitutions in one or both of the gene pair of the gene, and (2) conditions where the gene expression is suppressed by the action of nucleic acids having effects of suppressing chondromodulin-I gene expression (for example, antisense RNA or siRNA).

"Suppressed" in the present invention includes cases in which the chondromodulin-I gene expression is completely suppressed, and cases in which the level of chondromodulin-I expression in the animals of the present invention is significantly decreased compared with that in a wild-type animal.

The condition of (1) mentioned above also includes cases in which the expression of only one of the genes in the gene pair of chondromodulin-I gene (hetero-knockout animal) is suppressed, but preferably expression of both genes in the gene pair of chondromodulin-I gene is suppressed (homo-knockout animal).

In the present invention, regions where gene modifications are present are not particularly limited so long as they are regions where the gene expression can be suppressed, and examples include exon regions and promoter regions.

The gene knockout animals of the present invention can be prepared by those skilled in the art using generally known genetic engineering techniques. Gene knockout mice can be prepared as follows, for example. First, a DNA comprising an exon region of chondromodulin-I gene of the present invention is isolated from mice, and a suitable marker gene is inserted into this DNA fragment to construct a targeting vector. This targeting vector is introduced into a mouse ES cell line by the electroporation method or the like, and cell lines that have undergone homologous recombination are selected. Marker genes to be inserted are preferably antibiotic-resistant genes such as neomycin-resistant gene. When an antibiotic-resistant gene is inserted, cell lines that have undergone homologous recombination can be selected just by culturing them in a medium containing the antibiotic. Alternatively, the thymidine kinase gene and such can be linked to the targeting vector for more efficient selection. This allows elimination of cell lines that have undergone non-homologous recombination. It is also possible to efficiently obtain cell lines in which one member in the gene pair of a gene of the present invention has been inactivated, by assaying homologous recombinants by PCR and Southern blotting.

When selecting cell lines in which homologous recombination has taken place, it is preferable to use multiple clones to generate chimera since there is a risk of unknown gene destruction in sites other than the site of homologous recombination due to gene insertion. Chimeric mice can be obtained by injecting the obtained ES cell lines into mouse blastoderms. By crossing these chimeric mice, mice in which one member in the gene pair of the chondromodulin-I gene of the present invention is inactivated can be obtained. Further, by crossing these mice, mice in which both members in the gene pair of the gene of the present invention are inactivated can be obtained. Genetic modification can also be made in non-mouse animals from which ES cells are established by using similar procedures.

The above-mentioned knockout animals of the present invention are preferably knockout (knockdown) animals in which chondromodulin-I gene expression is suppressed by introducing into non-human animals nucleic acids having effects of suppressing chondromodulin-I gene expression (antisense RNAs, siRNAs, shRNAs, or such).

The above-mentioned knockdown animals can also be constructed by introducing non-human animals with a vector structured such that the nucleic acids (antisense RNAs, siRNAs, shRNAs, or such) of the present invention can be expressed.

The type of knockout animals of the present invention is not particularly limited so long as they are non-human animals, but they are usually higher-order animals, preferably mammals and more preferably primates. More specifically, animals of the present invention are preferably monkeys or rodents (order Rodentia) such as mice, rats, or hamsters, and more preferably mice.

In a preferred embodiment, the gene knockout (knockdown) non-human animals of the present invention are animals showing abnormality in their cardiac valves. This "abnormality" specifically refers to thickening, calcification, and decreased flexibility of the cardiac valves; vascular invasion or invasion of vascular endothelial cells and macrophages into the valvular tissues; turbulent blood flow in the aorta; pressure gradient between left ventricle to aorta; and such.

Furthermore, the above-mentioned non-human animals of the present invention can be used, for example, in the methods of screening for pharmaceutical agents of the present invention. More specifically, the present inventors discovered that the above-mentioned non-human animals can be used suitably, for example, in the screening for therapeutic agents for angiogenesis-related diseases (novel use). Therefore, in a preferred embodiment, the non-human animals of the present invention are animals for the screening methods to be described later.

The present invention also provides methods of screening for pharmaceutical agents of the present invention (angiogenesis-suppressing agents and therapeutic agents for angiogenesis-related diseases), which use the expression level of a chondromodulin-I gene as an indicator.

Substances that increase (enhance) the chondromodulin-I gene expression level are expected to become pharmaceutical agents of the present invention. Candidate compounds for angiogenesis-suppressing agents or therapeutic agents for angiogenesis-related diseases can be obtained efficiently by the screening methods of the present invention.

In a preferred embodiment, the methods of the present invention are methods of screening for pharmaceutical agents of the present invention (angiogenesis-suppressing agents and therapeutic agents for angiogenesis-related diseases), which comprise the steps of:
(a) contacting a test compound with cells expressing a chondromodulin-I protein;
(b) measuring expression level of the chondromodulin-I protein in the cells; and
(c) selecting a compound that increases the expression level compared with that measured in the absence of the test compound.

In the above-mentioned methods of the present invention, first, a test compound is contacted with cells expressing a chondromodulin-I protein (gene).

"Cells" that are used in this method are not particularly limited, but are preferably human-derived cells. Cells expressing an endogenous chondromodulin-I protein, or cells expressing a foreign chondromodulin-I gene that has been introduced can be used as the "cells expressing a chondromodulin-I protein". Normally, cells expressing a foreign chondromodulin-I gene can be prepared by introducing into host cells an expression vector inserted with a chondromodulin-I gene. Such expression vectors can be prepared using conventional genetic engineering techniques.

The test compounds subjected to the screening methods of the present invention are not particularly limited. Examples of such compounds include single compounds such as natural compounds, organic compounds, inorganic compounds, proteins, and peptides, as well as chemical libraries, expression products of gene libraries, cell extracts, cell culture supernatants, microzyme products, marine organism extracts, and plant extracts, but are not limited thereto.

These test compounds can be used by appropriately labeling them if necessary. Examples of the labels include radioactive labels and fluorescent labels.

Typically, a test compound is "contacted" with cells expressing a chondromodulin-I protein (gene) by adding the test compound to a culture medium of the cells expressing a chondromodulin-I protein, but the methods are not limited thereto. When the test compound is a protein or such, the "contact" can be achieved by introducing into the cells a DNA vector that expresses the protein.

As the next step in these methods, the expression level of the chondromodulin-I protein is determined. Herein, "protein expression" refers to both transcription and translation. The expression level can be determined using methods known to those skilled in the art.

The transcriptional level of the gene can be determined by, for example, extracting mRNAs from cells expressing a chondromodulin-I protein according to conventional methods, and carrying out Northern hybridization, RT-PCR, the DNA array method, or such using this mRNA as a template. Alternatively, the translational level of the gene can be determined by collecting protein fractions from the cells expressing the chondromodulin-I protein, and then detecting expression of the chondromodulin-I protein using an electrophoresis method such as SDS-PAGE. Furthermore, the translational level of the gene can also be determined by detecting expression of the chondromodulin-I protein by Western blot analysis using antibodies against the protein. The types of antibodies used for chondromodulin-I protein detection are not particularly limited so long as the gene can be detected, and for example, both monoclonal and polyclonal antibodies can be used.

The antibodies that bind to chondromodulin-I protein can be prepared by methods known to those skilled in the art. When the antibodies are polyclonal antibodies, they can be obtained, for example, using the following methods. Serum is obtained by immunizing small animals such as rabbits with a natural chondromodulin-I protein, or a recombinant chondromodulin-I protein expressed as a fusion protein, or a partial peptide thereof, with GST in microorganisms such as *Escherichia coli.* Antibodies are purified and prepared from the serum using, for example, ammonium sulfate precipitation, protein A columns, protein G columns, DEAE ion-exchange chromatography, or affinity columns on to which chondromodulin-I protein or a synthetic peptide is coupled. Alternatively, monoclonal antibodies can be produced by, for example, immunizing small animals such as mice with a chondromodulin-I protein or a partial peptide thereof, removing the spleen from each mouse, gently grinding the spleens to separate cells, fusing the cells with mouse myeloma cells using a reagent such as polyethylene glycol, and then selecting a clone that produces antibody that binds to the chondromodulin-I protein from the prepared hybridomas. Next, the obtained hybridoma is then transplanted into a mouse peritoneal cavity and ascites are collected from the mouse. The monoclonal antibody thus obtained can be prepared by purifying it using, for example, ammonium sulfate precipitation, protein A columns, protein G columns, DEAE ion-exchange chromatography, affinity columns on to which chondromodulin-I protein or a synthetic peptide is coupled.

Next in the present methods, compounds that increase the expression level of the chondromodulin-I protein compared with that measured when the test compounds are not contacted (that measured in the absence of the test compounds) are selected. Alternatively, the above-mentioned selection of (c) can be carried out by comparison with the control.

Compounds isolated by the present method have effects of suppressing angiogenesis (for example, angiogenesis in cardiac valves, retina, or such), and are expected to be therapeutic agents for angiogenesis-related diseases.

Other embodiments of the screening methods of the present invention are methods that use the chondromodulin-I protein activity (function) as an indicator. The above-mentioned methods are, for example, methods of screening for pharmaceutical agents of the present invention, which comprise the steps of:
(a) contacting a test compound with a chondromodulin-I protein, or cells or an extract solution of cells expressing the protein;
(b) measuring activity of the protein; and
(c) selecting a compound that increases the protein activity compared with that measured in the absence of the test compound.

In the present method, first, test compounds are contacted with a chondromodulin-I protein, or cells or an extract solution of cells expressing this protein.

Next, activity of the chondromodulin-I protein is measured. Examples of chondromodulin-I protein activity include the various activities (functions) described above. These activities can be appropriately measured by those skilled in the art using known methods or by referring to the various documents referred to in the present description.

Furthermore, compounds that increase (enhance) the protein activity compared with that when the test compounds are not contacted (control) are selected.

Other embodiments of the screening methods of the present invention are methods of selecting compounds that increase the expression level of the chondromodulin-I proteins (genes) of the present invention using reporter gene expression as an indicator.

Preferred embodiments of the above-mentioned methods of the present invention are methods of screening for pharmaceutical agents of the present invention, which comprise the steps of:
(a) contacting a test compound with cells or an extract solution of cells comprising a DNA structured such that a reporter gene is operably linked to the transcriptional regulatory region of a chondromodulin-I gene;
(b) measuring expression level of the reporter gene; and
(c) selecting a compound that increases the expression level compared with that measured in the absence of the test compound.

In these methods, test compounds are first contacted with cells or an extract solution of cells that comprise a DNA having a structure in which a reporter gene is operably linked to the transcriptional regulatory region of a chondromodulin-I gene. Herein, "operably linked" means that the transcriptional regulatory region of a chondromodulin-I gene is linked to a reporter gene in such a way as to induce reporter gene expression when a transcriptional factor binds to the transcriptional regulatory region of the chondromodulin-I gene. Thus, even when the reporter gene is connected to another gene and forms a fusion protein with the gene product, such a connection is included in the meaning of "operably linked", so long as the expression of the fusion protein is induced when the transcriptional factor binds to the transcriptional regulatory region of the chondromodulin-I gene. Those skilled in the art can obtain, using known methods, transcriptional regulatory regions for chondromodulin-I genes present in a genome, based on cDNA nucleotide sequences of chondromodulin-I genes.

The type of reporter gene used in the methods is not particularly limited, so long as its expression can be detected, and examples include the CAT gene, lacZ gene, luciferase gene, and GFP gene. "Cells that comprise a DNA structured such that a reporter gene is operably linked to the transcriptional regulatory region of a chondromodulin-I gene" include, for example, cells introduced with a vector inserted with such structure. Those skilled in the art can prepare such vectors using known methods. Such a vector can be introduced into cells using conventional methods, for example, calcium phosphate precipitation, electroporation, lipofection method, and microinjection. "Cells that comprise a DNA structured such that a reporter gene is operably linked to the transcriptional regulatory region of a chondromodulin-I gene" also include cells in which that structure has been inserted into their chromosome. The DNA structures can be inserted into chromosomes using methods generally used by those skilled in the art, for example, gene transfer methods using homologous recombination.

"Extract solution of cells that comprise a DNA structured such that a reporter gene is operably linked to the transcriptional regulatory region of a chondromodulin-I gene" include, for example, mixtures prepared by adding a DNA structured such that a reporter gene is operably linked to the transcriptional regulatory region of a chondromodulin-I gene, to cell extract solutions included in commercially available kits for *in vitro* transcription and translation.

In these methods, "contact" can be achieved by adding test compounds to the culture media of "cells that comprise a DNA structured such that a reporter gene is operably linked to the transcriptional regulatory region of a chondromodulin-I gene", or by adding test compounds to the above-mentioned commercially available cell extract solutions which comprise such DNA. When the test compounds are proteins, the "contact" can also be achieved, for example, by introducing the cells with a DNA vector that expresses those proteins.

The next step in these methods is determining the level of reporter gene expression. The expression level of a reporter gene can be determined depending on the type of the reporter gene, by methods known to those skilled in the art. For example, when the reporter gene is a CAT gene, its expression level can be determined by detecting the acetylation of chloramphenicol, which is mediated by the CAT gene product. When the reporter gene is a lacZ gene, its expression level can be determined by detecting color development in a chromogenic compound, mediated by the catalytic action of the lacZ gene expression product. When the reporter gene is a luciferase gene, the expression level can be determined by detecting the fluorescence of a fluorescent compound, mediated by the catalytic action of the luciferase gene expression product. Alternatively, when the reporter gene is a GFP gene, the expression level can be determined by detecting the fluorescence of the GFP protein.

Next, in the present methods, compounds that increase (enhance) the measured expression level of the reporter gene compared with that measured in the absence of the test compound are selected.

The present invention also relates to methods of using the above-mentioned animals of the present invention to screen for pharmaceutical agents (therapeutic agents for angiogenesis-related diseases and angiogenesis-suppressing agents) of the present invention.

The methods of the present invention are, for example, methods of screening for pharmaceutical agents of the present invention, which comprise the steps of:
(a) administering a test compound to a gene knockout non-human animal of the present invention;
(b) measuring expression level or activity of chondromodulin-I protein in the gene knockout non-human animal; and
(c) selecting a compound that increases the expression level or activity of chondromodulin-I protein compared with when the test compound is not administered.

First, a test compound is administered to the gene knockout non-human animals mentioned above. The test compound can be administered by oral or parenteral administration, but preferably by parenteral administration, and the specific examples include injections, transnasal administrations, transpulmonary administrations, and transdermal administrations. Examples of injections include intravenous injection, intramuscular injection, intraperitoneal injection, and subcutaneous injection, and the administration may be systemic or local (for example, cardiac valves, or retina).

When the test compounds are DNAs, they can be administered into a living body by viral vectors such as retroviruses, adenoviruses, and Sendai viruses and by non-viral vectors such as liposomes. Examples of administration methods include *in vivo* and *ex vivo* methods.

Next, in the present methods, the expression level or activity of chondromodulin-I protein in the gene knockout animals is measured. More specifically, the expression level or activity of chondromodulin-I protein in tissues, organs, cells, or such (preferably cardiac valves, interior of eyes, retina, or such) from these animals is measured. Expression level and activity measurements can be performed by the methods described above.

Then, in the present methods, compounds that increase the expression level or activity of chondromodulin-I protein compared with when the test substance is not administered (control) are selected.

The screening of the present invention can also be carried out with gene knockout animals of the present invention, using morphological change of cardiac valves or retina from these animals as an indicator.

Preferred embodiments of the present invention are methods of screening for pharmaceutical agents of the present invention, which comprise the steps of:
(a) administering a test compound to a gene knockout non-human animal of the present invention;
(b) observing the cardiac valve or retina in the gene knockout non-human animal; and
(c) selecting a compound that normalizes the cardiac valve or retina compared with when the test compound is not administered (control).

Next, in the present methods, phenotype-dependent morphological changes exhibited by non-human animals whose chondromodulin-I gene has been knocked out are observed. More specifically, candidate compounds for pharmaceutical agents of the present invention can be obtained by selecting substances that normalize abnormal cardiac valves or retina in the knockout animals. For example, compounds that normalize thickening, calcification, and such in the cardiac valves of the knockout non-human animals are selected.

The present invention also provides kits containing various pharmaceutical agents, reagents, and such to be used in the screening methods of the present invention.

For a kit of the present invention, for example, reagents can be suitably selected from the various reagents of the present invention described above according to the screening methods. For example, a kit of the present invention contains as a component, a probe for a chondromodulin-I gene, an oligonucleotide such as a primer for amplifying any region of that gene, an antibody that recognizes a chondromodulin-I protein (anti-chondromodulin-I protein antibody), or such that can be used to detect the chondromodulin-I protein.

The above-mentioned oligonucleotide is, for example, an oligonucleotide that specifically hybridizes to the DNA of a chondromodulin-I gene of the present invention. Herein, "specifically hybridizes" means that under ordinary hybridization conditions, and preferably under stringent hybridization conditions (for example the conditions described in Sambrook et al., Molecular Cloning, Cold Spring Harbor Laboratory Press, New York, USA, 2nd edition, 1989), cross hybridization with DNAs encoding other proteins does not take place significantly. So long as specific hybridization is possible, the oligonucleotide does not have to be completely complementary to the nucleotide sequence of the chondromodulin-I gene.

In the present invention, examples of hybridization conditions include conditions such as "2x SSC, 0.1% SDS, 50°C", "2x SSC, 0.1% SDS, 42°C", and "1x SSC, 0.1% SDS, 37°C", and more stringent conditions such as "2x SSC, 0.1% SDS, 65°C", "0.5x SSC, 0.1% SDS, 42°C", and "0.2x SSC, 0.1% SDS, 65°C". More specifically, a method using Rapid-hyb buffer (Amersham Life Science) involves conducting prehybridization at 68°C for 30 minutes or longer, adding a probe, allowing hybrid formation by maintaining the temperature at 68°C for one hour or longer, washing three times in 2x SSC, 0.1% SDS at room temperature for 20 minutes, then washing three times in 1x SSC, 0.1% SDS at 37°C for 20 minutes, and finally washing twice in 1x SSC, 0.1% SDS at 50°C for 20 minutes. Alternatively, for example, one may conduct prehybridization at 55°C for 30 minutes or longer in Expresshyb Hybridization Solution (CLONTECH), add a labeled probe and incubate at 37°C to 55°C for one hour or longer, wash three times in 2x SSC, 0.1% SDS at room temperature for 20 minutes, and then wash once in 1x SSC, 0.1% SDS at 37°C for 20 minutes. Herein, conditions of higher stringency can be produced, for example, by setting a higher temperature for prehybridization, hybridization, or second wash. For example, the temperature for prehybridization and hybridization can be set to 60°C, or to 68°C for higher stringency. In addition to these conditions such as buffer salt concentration and temperatures, those skilled in the art can set the conditions by taking into account other conditions such as probe concentration, probe length, nucleotide sequence composition of the probe, and reaction time.

The oligonucleotides can be used as probes or primers in the above-mentioned kits for screening in the present invention. When using the oligonucleotide as a primer, its length is normally 15 bp to 100 bp, and preferably 17 bp to 30 bp. The primer is not particularly limited so long as at least part of a DNA of the chondromodulin-I gene of the present invention can be amplified.

Various reagents, containers, and such used in the methods of the present invention can also be included in the kits of the present invention. For example, various reaction reagents, cells, culture media, control samples, buffers, instructions indicating methods of use, and such can be appropriately included.

Furthermore, the present invention provides artificial heart valves comprising the following (a) and (b) as major components:
(a) cells expressing a chondromodulin-I gene; and
(b) a decellularized valve or biodegradable high-molecular compound.

The artificial heart valves of the present invention are hybrid-type artificial heart valves comprising the above (a) and (b) as major components, and they may be referred to as, for example, "hybrid-type regenerative valve(s)".

Furthermore, the present invention provides artificial heart valves produced by the steps of:
(a) culturing and growing cells that express a chondromodulin-I gene;
(b) seeding the cells of (a) onto the surface of a decellularized valve or biodegradable high-molecular compound; and
(c) culturing until the cells expressing the chondromodulin-I gene fill the pore spaces of the decellularized valve or biodegradable high-molecular compound of (b).

In this production process, first, cells expressing a chondromodulin-I gene are cultured. Cells expressing the chondromodulin-I gene can be obtained by biopsy, for example, by excising a part of a (cardiac) valvular tissue (for example, heart valve ring tissue or tricuspid valve) from a test subject or an individual who is not a subject being tested, and then removing the endothelial cells on the surface. The obtained cells are explant cultured and grown until the volume reaches a sufficient level. Culture conditions generally known to those skilled in the art, for example, culture conditions described in the section of cell culturing of the paper (Lester W., Rosenthal A., Granton B., Gotlieb A. I. Porcine mitral valve interstitial cells in culture. Lab. Invest. 59, 710-719 (1988)) can be used for the explant culture conditions.

Next the cells are seeded onto the surface of decellularized valves or biodegradable high-molecular compounds. The decellularized valves and biodegradable high-molecular compounds used are preferably those having pore spaces, more specifically those that are porous.

Then, culturing is continued until the cells seeded on the surface infiltrate the pore spaces (pores) and fill the pore spaces present in the decellularized valves or biodegradable high-molecular compounds. Conditions already described in the paper (Lester W., Rosenthal A., Granton B., Gotlieb A. I. Porcine mitral valve interstitial cells in culture. Lab. Invest. 59, 710-719 (1988)), for example, can be used for the culturing conditions.

When the cells have sufficiently filled the above-mentioned pore spaces (pores), the decellularized valves or biodegradable high-molecular compounds containing the cells are switched to a serum-free culture medium.

Artificial heart valves of the present invention produced in this manner are washed with physiological saline to remove serum components, and then can be used to perform a valve replacement operation on patients needing replacement of their valve in which destruction has progressed.

The above-mentioned "cells expressing the chondromodulin-I gene" of the present invention are preferably valvular interstitial cells derived from a test subject or from an individual who is not a test subject. More specifically, self valvular interstitial cells or valvular interstitial cells from a different individual (third person) can be used.

The above-mentioned "decellularized valve(s)" of the present invention refers to a tissue valve that has been decellularized (cellular components have been removed). Decellularization can be performed by methods known to those skilled in the art. Furthermore, as described above, decellularized valves of the present invention are preferably porous.

As the "tissue valve(s)" in the present invention, a porcine tissue valve, for example, can be used.

Examples of the "biodegradable high-molecular compound(s)" of the present invention include polylactic acid. As mentioned above, biodegradable high-molecular compounds of the present invention are preferably porous.

The present inventors revealed that valvular interstitial cells secrete chondromodulin-I, which is an angiogenesis-suppressing factor, to prevent infiltration of new blood vessels into valvular interstitial tissues, and protect the valvular tissues from destruction due to accompanying inflammatory cell infiltration and MMP activation. So far, the function of hybrid-type regenerative valves prepared by adding bone marrow cells or such to porcine tissue valves or biodegradable high-molecular compounds (for example, polylactic acid) could not be maintained for a long time due to accompanying vascular invasion and infiltration of inflammatory cells into valvular tissues. Accordingly, the present inventors tried seeding self valvular interstitial cells or valvular interstitial cells from a different individual (a third person) to porcine tissue valves (decellularized valves) or biodegradable high-molecular compounds (for example, polylactic acid) to prepare hybrid-type regenerative valves that maintain tissues having a structure similar to that of the original valve tissues, and use them in clinical applications.

More specifically, the present invention provides artificial heart valves having the function of suppressing angiogenesis in valvular tissues.

Furthermore, the present invention relates to methods for producing angiogenesis-suppressing agents and therapeutic agents for angiogenesis-related diseases which comprise the step of mixing (combining) any of the following (a) to (c) with pharmaceutically acceptable carriers or vehicles:
(a) a chondromodulin-I protein (for example, the human chondromodulin-I protein as shown in the amino acid sequence of SEQ ID NO: 2);
(b) a protein functionally equivalent to a chondromodulin-I protein, which comprises an amino acid sequence with one or more amino acid deletions, substitutions, or additions in the amino acid sequence of a chondromodulin-I protein (for example, the human chondromodulin-I protein amino acid sequence of SEQ ID NO: 2); and
(c) a DNA encoding the protein of (a) or (b) above.

"Pharmaceutically acceptable carriers or vehicles" refer to materials that can be administered with any of the aforementioned (a) to (c) and do not significantly inhibit the angiogenesis-suppressing effects. Examples of such carriers and vehicles include deionized water, sterilized water, sodium chloride solution, dextrose solution, dextrose and sodium chloride, Ringer solution containing lactic acid, culture medium, serum, and phosphate buffered saline (PBS), and these may be combined appropriately with any of the above-mentioned (a) to (c) for formulation. This may also be concentrated by centrifugation and re-suspended in a physiological solution such as physiological saline if necessary. Stabilizers for liposome membranes (for example, sterols such as cholesterol) may also be included. Antioxidants (for example, tocopherol or vitamin E) may also be included. In addition, plant oils, suspending agents, surfactants, stabilizers, biocides, and such may also be included. Furthermore, preservatives and other additives can be added. The compositions of the present invention may be in the form of aqueous solution, capsule, suspension, syrup, or such.

The present invention further relates to methods for preventing or treating angiogenesis-related diseases, which comprise the step of administering any of the aforementioned (a) to (c) to individuals (for example, patients). The individuals in the preventive or therapeutic methods of the present invention are preferably human, but are not particularly limited thereto, and may be non-human animals. The dosage of any of the aforementioned (a) to (c) of the present invention differs depending on the disease, the body weight, age, sex, and symptoms of the patient, purpose of administration, form of the composition to be administered, method of administration, and such, but it can be determined appropriately by those skilled in the art. The route of administration can be selected appropriately, and it may be, for example, transdermal, intranasal, transbronchial, intramuscular, intraperitoneal, intravenous, intraarticular, or subcutaneous. The administration may be systemic or local. When administering to non-human animals, the amount of administration can be converted from the dosage for human, for example, based on the ratio of body weight or volume ratio of a target site of administration (for example, average values) between the animal of interest and human.

The present invention also relates to methods for treating angiogenesis-related diseases which comprise the step of using the above-mentioned artificial heart valves of the present invention.

The present invention further provides use of the substance of any one of the above-mentioned (a) to (c) in the production of angiogenesis-suppressing agents and therapeutic agents for angiogenesis-related diseases.

All prior art references cited herein are incorporated by reference into this description.

### Examples

Herein below, the present invention will be specifically described with reference to Examples, but it is not to be construed as being limited thereto.

### [Example 1] Preparation of human samples

From patients who had undergone valve replacement operation for valvular stenosis or regurgitation (29 males; average age of 60.3 ± 17.7 years), 18 aortic valve samples and 11 mitral valve samples were collected. Immediately after sample isolation, the samples were fixed in formaldehyde and embedded in paraffin. Three aortic and mitral valves which seemed non-calcified, smooth, and flexible, and normal by microscopic and visual inspection were obtained from three autopsy patients (average age of 44.3 ± 9.7 years) as controls.

### [Example 2] Preparation of mice and rats

Wild-type ICR strain mice and Wistar strain rats were purchased from CLEA Japan (Tokyo, Japan). ApoE^{-/-} mice (Plump, A. S. et al. Severe hypercholesterolemia and atherosclerosis in apolipoprotein E-deficient mice created by homologous recombination in ES cells. Cell 71, 343-53 (1992)) were purchased from Jackson Laboratories, and were given normal feed until they reached a sufficiently old age (n = 5, 90.5 ± 3.7 weeks old). The chm-I^{-/-} strain was prepared by known methods (Nakamichi, Y. et al. Chondromodulin I is a bone remodeling factor. Mol. Cell Biol. 23, 636-44 (2003)) and maintained until it became 90.2 ± 7.4 weeks old (n = 7).

### [Example 3] Isolation of adult rat aortic valvular interstitial cells (VICs)

Five-week-old Wistar strain rats were anesthetized and their hearts were removed. Lobules of the aortic valves were quickly collected, cut into small pieces under a stereoscopic microscope, and used for explant culture according to a known method (Lester W, R. A., Granton B, et al. Porcine mitral valve interstitial cells in culture. Lab. Invest. 710-719 (1988)). Small pieces of tissue (5 x 5 mm) were removed, placed onto 12-well collagen-coated plates (Iwaki, Tokyo, Japan), and grown in M199 (Sigma-Aldrich, Tokyo, Japan) containing 10% fetal bovine serum (FBS). Conditioned medium (CM) was obtained from confluent valvular interstitial cells three days after medium change, and used for further analysis.

### [Example 4] Preparation of cell culture

NIH3T3 cells were maintained by known methods (Hisaka, Yet al. Powerful and controllable angiogenesis by using gene-modified cells expressing human hepatocyte growth factor and thymidine kinase. J. Am. Coll. Cardiol. 43, 1915-22 (2004)). Human coronary artery endothelial cells (HCAECs) were purchased from TaKaRa Biotechnology (Tokyo, Japan) and maintained using a known method (Hamilton, K. L., Mbai, F. N., Gupta, S. & Knowlton, A. A. Estrogen, heat shock proteins, and NFkappaB in human vascular endothelium. Arterioscler. Thromb. Vasc. Biol. 24, 1628-33 (2004)). Cells that were passaged three to five times were used in the experiments.

### [Example 5] Analysis by reverse transcription PCR (RT-PCR)

Total RNAs were isolated using TRIzo1 (Gibco-BRL) and treated with DNase I (Roche). RT-PCR was performed by known methods (Enomoto, H. et al. Vascular endothelial growth factor isoforms and their receptors are expressed in human osteoarthritic cartilage. Am. J. Pathol. 162, 171-81 (2003)) using the following primers:
Mouse chm-I (Genbank^{™} Accession No. NM_010701): (forward)
   5'-CTTAAGCCCATGTATCCAAA-3' / SEQ ID NO: 3, (reverse)
   3'-CCAGTGGTTCACAGATCTTC-5' / SEQ ID NO: 4; gapdh (forward)
   5'-TTCAACGGCACAGTCAAGG-3' / SEQ ID NO: 5, (reverse)
   3'-CATGGACTGTGGTCATGAG-5' / SEQ ID NO: 6.
RT-PCR was performed for chm-I using various organs. Cartilage and eye were used as positive controls. chm-I was not detected in organs besides the heart (Fig. 1a). chm-I was strongly expressed in the cardiac valves, but not expressed in the atria and ventricles (Fig. 1b). During rat embryogenesis, chm-I expression appeared for the first time in the heart at E9.5, and continued until adulthood (Fig. 1c).

### [Example 6] Analysis by real-time quantitative RT-PCR (QT-PCR)

The relative amount of rat chm-I mRNA in the rat heart was assessed by TaqMan real-time PCR using the ABI PRISM 7700 Sequence Detection System (PE Applied Biosystems) according to a known method (Aoyama, T. et al. Expression of the chondromodulin-I gene in chondrosarcomas. Cancer Lett. 204, 61-8 (2004)). A 75-bp fragment from +411 (exon 4) to +485 (exon 5) of the chm-I cDNA (Genbank^{™} Accession No. NM_030854) was amplified using specific primers (sense, 5'-GAAGGCTCGTATTCCTGAGGTG-3' /SEQ ID NO: 7; and antisense 5'-TGGCATGATCTTGCCTTCCAGT-3' / SEQ ID NO: 8) and labeled with a TaqMan probe (5'-FAM-CGTGACCAAACAGAGCATCTCCTCCA-3'-TAMRA / SEQ ID NO: 9). GAPDH mRNA was used as the internal control, and all reactions were run in triplicate per sample. The ratio of chm-I/GAPDH in each sample was calculated, and the expression level of chm-I gene was determined as a relative value using the chm-I/GAPDH ratio in whole rat eye as a standard (1.0). The same analysis was performed three times, and the level was expressed as a percentage of mRNA level in comparison with the whole rat eye mRNA level standardized to the GAPDH level.

Quantitative PCR revealed that chm-I expression is 800 times higher in the cardiac valves than in the atria and ventricles (Fig. 1d).

### [Example 7] Western blotting analysis

Wistar strain rat and human autopsy tissues (atria, ventricles, cardiac valves, and cartilages) were homogenized in a lysis buffer (20 mM Tris (pH7.4), 1 mM EDTA, 1 mM EGTA, and complete mini^{®} (Roche, Germany) tablet /10 mL of buffer). Western blot analysis was carried out using a known method (Funaki, H. et al. Expression and localization of angiogenic inhibitory factor, chondromodulin-I, in adult rat eye. Invest. Ophthalmol. Vis. Sci. 42, 1193-200 (2001)). To visualize the ChM-I protein, the loading level of each sample was varied. The membrane was incubated together with a polyclonal rabbit anti-ChM-I antibody at 4°C overnight. The membrane was incubated with a horseradish peroxidase (HRP)-conjugated anti-rabbit IgG antibody (Amersham Pharmacia Biotech, Piscataway, New Jersey), and signals were visualized using SuperSignal West Pico (PIERCE) according to the instructions provided by the supplier.

Western blotting showed that ChM-I was present in the rat and human cardiac valves (Figs. 1e and f). The 25-kDa ChM-I protein found in the cardiac valves was assumed to be the mature glycosylated form detected similarly in cartilage extracts. Atrial and ventricular extracts did not show signals that indicate the presence of ChM-1.

### [Example 8] Analyses by in situ hybridization and immunohistochemistry

### (In situ hybridization)

Paraffin-embedded sections were treated with proteinase K, and *in situ* hybridization was performed by a known method (Enomoto, H. et al. Vascular endothelial growth factor isoforms and their receptors are expressed in human osteoarthritic cartilage. Am. J. Pathol. 162, 171-81 (2003)). The template DNA was a 879-bp cDNA encoding a mouse chm-I cloned into a pCR II-TOPO vector. Colors were developed using a solution of 0.2 mg/mL 3,3'-diaminobenzidine tetrahydrochloride in 50 mmol/L Tris-HCl (pH7.6) containing 0.003% hydrogen peroxide. The sections were counterstained with hematoxylin and observed under a microscope.

Furthermore, 9.0-, 9.5-, 10.0-, and 12.5-day mouse embryos (E) were collected, and *whole-mount in situ* hybridization was performed using a DIG-labeled RNA probe by a known method (Dietz, U. H., Ziegelmeier, G., Bittner, K., Bruckner, P. & Balling, R. Spatio-temporal distribution of chondromodulin-I mRNA in the chicken embryo: expression during cartilage development and formation of the heart and eye. Dev. Dyn. 216, 233-43 (1999)).

### (Immunohistochemistry and immunofluorescent staining)

Hearts from pregnant or non-pregnant adult mice were perfused through the apex of the hearts with PBS, fixed with 4% paraformaldehyde (PFA)/PBS, and these were used for immunostaining according to a known method (Funaki, H. et al. Expression and localization of angiogenic inhibitory factor, chondromodulin-I, in adult rat eye. Invest. Ophthalmol. Vis. Sci. 42, 1193-200 (2001)). More specifically, hearts of pregnant or non-pregnant adult mice were dissected, fixed by soaking in 4% PFA overnight at 4°C, and then embedded in paraffin. Before applying the primary antibody, paraffin was removed from the sections in xylene. The sections were placed in a pH6.0 10 mmol citric acid monohydrate (DAKO, Glostrup, Denmark) and heated for three minutes in a microwave oven. The sections were rinsed with PBS, and then incubated overnight at 4°C with 5% normal rabbit serum, an affinity-purified anti-polyclonal rabbit anti-mouse ChM-I antibody, a polyclonal rabbit anti-VEGF-A antibody (200-fold dilution; sc-507; Santa Cruz Biotechnology, California), an anti-von Willebrand factor antibody (200-fold dilution; vWF; RB-281-A0; Lab Vision Corporation, Westinghouse Dr., Fremont, California), or an anti-MAC-1 antibody (200-fold dilution; 557394; BD PharMingen, Inc., San Diego, California). To observe the immunohistochemical reactions, signals were detected by applying as a pigment-producing substrate a 0.05% 3,3'-diaminobenzidine tetrahydrochloride (Sigma-Aldrich, Tokyo, Japan) in 0.05 M Tris buffered saline solution (pH7.6) containing 0.01 % hydrogen peroxide. The sections were counterstained with hematoxylin, subjected to stepwise ethanol dehydration, and then enclosed in Permount (Fisher Scientific, Pittsburgh, Pennsylvania) (Hiraki, Y. et al. Molecular cloning of human chondromodulin-I, a cartilage-derived growth modulating factor, and its expression in Chinese hamster ovary cells. Eur. J. Biochem. 260, 869-78 (1999)).

For immunofluorescence assay, the sections were incubated with a secondary antibody conjugated to Alexa 488 or 594 (Molecular Probes, Eugene, Oregon). The nuclei were stained with TOTO-3 (Molecular Probes, Eugene, Oregon). The slide glasses were observed under a confocal laser scanning microscope (LSM 510 META; Carl Zeiss, Jena, Germany). Optical sections were obtained at a resolution of 1024 x 1024 pixels and analyzed using the LSM software (Carl Zeiss, Jena, Germany). As a negative control for each of the immunostaining experiments, the present inventors performed the experiments using a non-immunized rabbit serum as a primary antibody.

*In situ* hybridization (Fig. 8) and immunohistochemistry (Figs. 2 and 9) showed that ChM-I was present in all four cardiac valves in the adult mouse heart. The serial sections showed that ChM-I expression and VEGF-A expression appeared to be reciprocal. The ChM-I protein was detected in valvular interstitial cells (VICs) and the entire extracellular matrix, but it was not detected in the outer endothelial cell layer of cardiac valves.

During development, cardiac valve precursor cells from the atrioventricular cushions (AVCs) and outflow tract (OFT) expressed the chm-I transcripts and ChM-I protein from E9.5. The beginning of this expression matched the RT-PCR result. In E10.0 embryos, ChM-I was expressed in the cardiac jelly covering the trabeculating cardiomyocytes of the left ventricle (LV), the outer curvature of the right ventricle, and the outflow tract. ChM-I expression in the ventricles decreased gradually as development progressed; and by mid-embryogenesis, both the chm-I transcripts and protein disappeared. Difference in the localization of ChM-I and VEGF-A was apparent at all development stages. VEGF-A expression was limited to cardiomyocytes and endothelial cells facing the ventricular cavity, while ChM-I expression was limited to the primordia of valve lobules.

### [Example 9] Expression analysis of ChM-I and VEGF-A in valvular sclerosis in aged ApoE^{-/-} mice

The expression of anti-angiogenic ChM-I and angiogenic VEGF-A was investigated in the cardiac valves of aged ApoE^{-/-} mice (90.5 ± 3.7 weeks old), which are an atherosclerosis model with abnormal fat deposition and calcification in their cardiac valves. There was no ChM-I expression in the calcified regions (Figs. 3a, b, f, and h) of both aortic valve and mitral valve with newly-infiltrated VEGF-A-positive cells (Figs. 3g, l, k, and m). Age-matched wildtype mice (88.5 ± 4.4 weeks old) showed the expected physiological ChM-I-positive and VEGF-A-negative expression patterns, and did not show sclerosis or calcification. *In in situ* hybridization for chm-I, signals in the lobules of cirrhotic aortic valves were not detected in ApoE^{-/-} mice (Fig. 3d), while age-matched wildtype mice showed positive signals (Fig. 3e).

### [Example 10] Expression analysis of ChM-I and VEGF-A in pathological angiogenesis in human cardiac valves

By immunostaining autopsy samples and surgical specimen using both hematoxylin-eosin (HE) staining and Azan staining, expression profiles of ChM-I and VEGF-A in the cardiac valves of the valvular heart disease patients were determined (Fig. 4). Blood vessels were not found in the cardiac valves of the patients without VHD. In normal valves, ChM-I was detected in the fibrous layer, spongy layer, and ventricular layer, and was not present in the endothelial layer, while VEGF-A was not present in any of the cell layers. In contrast, cardiac valves from the patients with infective endocarditis (IE), rheumatic heart disease (RHD), and atherosclerosis had many blood vessels. ChM-I was significantly down-regulated in the new angiogenic regions strongly expressing VEGF-A, and this was consistent with the findings from ApoE^{-/-} mice. This expression profile was not observed in the cardiac valves of the patients with annuloaortic ectasia or ruptured mitral chordae tendineae (Table 1).

**Table 1**

| No. | Age | Sex | Valvular heart disease | Cause | Angiogenesis | VEGF | ChM-I | Calcification |
|---|---|---|---|---|---|---|---|---|
| 1 | 56 | M | Aortic stenosis | Atherosclerosis | +++ | +++ | + | +++ |
| 2 | 54 | M | Aortic stenosis | Atherosclerosis | +++ | +++ | ± | ++ |
| 3 | 74 | M | Aortic stenosis | Atherosclerosis | ++ | +++ | + | +++ |
| 4 | 76 | F | Aortic stenosis | Atherosclerosis | +++ | +++ | ± | +++ |
| 5 | 51 | M | Aortic regurgitation | Atherosclerosis | +++ | ++ | + | +++ |
| 6 | 69 | F | Aortic regurgitation | Atherosclerosis | ++ | + | ++ | ++ |
| 7 | 76 | M | Aortic regurgitation | Bicuspid valve | +++ | ++ | + | ++ |
| 8 | 77 | M | Aortic stenosis | Bicuspid valve | +++ | ++ | + | +++ |
| 9 | 58 | M | Aortic stenosis | Bicuspid valve | + | +++ | ± | +++ |
| 10 | 72 | F | Aortic stenosis | Bicuspid valve | +++ | ++ | ± | +++ |
| 11 | 71 | M | Aortic stenosis | Bicuspid valve | ++ | +++ | + | ++ |
| 12 | 76 | F | Aortic stenosis | Bicuspid valve | + | ++ | + | ++ |
| 13 | 68 | F | Aortic regurgitation | Bicuspid valve | +++ | +++ | ++ | ++ |
| 14 | 73 | M | Aortic regurgitation | Bicuspid valve | ++ | ++ | + | ++ |
| 15 | 55 | M | Aortic regurgitation | Rheumatic heart disease | +++ | +++ | + | ++ |
| 16 | 58 | F | Aortic regurgitation | Rheumatic heart disease | +++ | +++ | + | ++ |
| 17 | 71 | F | Aortic regurgitation | Rheumatic heart disease | ++ | + | ± | +++ |
| 18 | 73 | M | Mitral stenosis | Rheumatic heart disease | +++ | +++ | ± | +++ |
| 19 | 35 | M | Aortic regurgitation | Infective endocarditis | +++ | +++ | ± | - |
| 20 | 37 | F | Mitral regurgitation | Infective endocarditis | ++ | ++ | ± | - |
| 21 | 60 | F | Mitral regurgitation | Infective endocarditis | +++ | ++ | + | + |
| 22 | 48 | M | Mitral regurgitation | Infective endocarditis | +++ | ++ | ± | - |
| 23 | 24 | M | Aortic regurgitation | Marfan syndrome | - | - | +++ | - |
| 24 | 45 | F | Aortic regurgitation | Annuloaortic ectasia | - | - | +++ | - |
| 25 | 60 | M | Aortic regurgitation | Annuloaortic ectasia | + | ++ | ++ | - |
| 26 | 72 | M | Aortic regurgitation | Noncoronary cusp prolapse | ++ | + | ++ | ++ |
| 27 | 40 | M | Mitral regurgitation | Mitral valve prolapse | - | - | +++ | - |
| 28 | 70 | F | Mitral regurgitation | Ruptured mitral chordae tendineae | + | + | ++ | ++ |
| 29 | 72 | M | Mitral regurgitation | Ruptured mitral chordae tendineae | - | - | +++ | - |
| 30 | 34 | M | No valvular heart disease | (Gerstmann's syndrome) | - | - | +++ | - |
| 31 | 50 | M | No valvular heart disease | (Subarachnoid hemorrhage) | - | - | +++ | - |
| 32 | 49 | F | No valvular heart disease | (Moyamoya disease) | - | - | +++ | - |

These findings clearly show that development of angiogenesis and cirrhotic changes in the diseased cardiac valves are directly related to ChM-I expression.

### [Example 11] Analysis of apoptosis induction and suppression of in vivo tube formation and migration in human coronary artery endothelial cells by ChM-I derived from valvular interstitial cells

### (Acetyl-LDL treatment)

Whether or not ChM-I is produced by valvular interstitial cells and whether or not VIC-derived ChM-I affects tube formation and growth of human coronary artery endothelial cells (HCAECs) *in vitro* were examined.

Rat primary-cultured cardiac valves and explant culture of valvular interstitial cells were prepared. Valvular interstitial cells and human coronary artery endothelial cells were treated with 10 µg/mL acetylated apoprotein (Ac-LDL) labeled with DiI (Molecular Probes, Eugene) at 37°C for one hour. The fluorescent cells were observed under a Nikon Diaphot microscope (excitation at 554 nm, emission at 571 nm).

The results are shown in Figs. 5a to f. The explant cells were a heterogenous population of cobblestone- and spindle-shaped cells, which are characteristic of valvular interstitial cells on day 3 (Zacks, S. et al. Characterization of Cobblestone mitral valve interstitial cells. Arch. Pathol. Lab. Med. 115, 774-9 (1991)). Seven days later, both cell types became more fibroblast-like and more elongated, and as already known, they formed an orthogonal overgrowth pattern after confluence (Lester W, R. A., Granton B, et al. Porcine mitral valve interstitial cells in culture. Lab. Invest. 710-719 (1988)). The cells were found to be negative for the acetyl LDL-DiI complex, and this was consistent with the cardiac valves containing valvular interstitial cells with an endothelial cell layer on the outer surface. Immunostaining showed that ChM-I was expressed in the cytoplasm of valvular interstitial cells and that ChM-I was not present in the negative control NIH3T3 cells.

### (Tube formation assay)

Twenty-four-well culture plates (Costar, Coming, New York) were coated with a growth factor-supplemented Matrigel (0.4 mL; Becton Dickinson Labware, Bedford, Massachusetts) and incubated at 37°C for thirty minutes. Four-hour-starved human coronary artery endothelial cells were treated with trypsin-EDTA, and then suspended for 20 minutes in a culture medium. The cells were seeded at a density of 10,000 cells/well in polymerized Matrigel in the presence or absence of the CM of valvular interstitial cells or NIH3T3 cells, and tube formation assays were performed as previously described (Oshima, Y et al. Expression and localization of tenomodulin, a transmembrane type chondromodulin-I-related angiogenesis inhibitor, in mouse eyes. Invest. Ophthalmol. Vis. Sci. 44, 1814-23 (2003)). After six hours of incubation at 37°C, photographs were taken with a phase-contrast light microscope (Carl Zeiss). To quantitatively evaluate the formation of capillary-like structures in the human coronary artery endothelial cells, the total length of the tube-like structures per visual field was measured using an image processing and analyzing software (Scion Image computer program, available from the public domain of National Institutes of Health (the United States), Bethesda, Maryland). Each experiment was performed five times.

As a result, human coronary artery endothelial cells formed capillary-like tube structures on the Matrigel six hours later (Fig. 5g), as reported previously (Oshima, Y et al. Expression and localization of tenomodulin, a transmembrane type chondromodulin-I-related angiogenesis inhibitor, in mouse eyes. Invest. Ophthalmol. Vis. Sci. 44, 1814-23 (2003)). After treating human coronary artery endothelial cells with the valvular interstitial cell-conditioned medium (CM), the capillary-like structures became less pronounced (Fig. 5i) compared with the control cells (Fig. 5g) and CM-treated NIH3T3 cells (Fig. 5h).

### (RNA interference)

Double-stranded small interference RNA (siRNA) against rat chm-I (chm-I-siRNA, 5'-AACCUCCUGGCAGUAGAUGUA-3' / SEQ ID NO: 10) or GL3 luciferase (GL3-luc-siRNA, 5'-CUUACGCUGAGUACUUCGA-3' / SEQ ID NO: 11) were used to transfect valvular interstitial cells grown to 90% confluence using Oligofectamine (Invitrogen). Three days after the transfection, conditioned medium from the cells was used for the experiment.

When valvular interstitial cells were treated with chm-I-specific siRNA, the chm-I transcription level significantly decreased (data not shown). Human coronary artery endothelial cells cultured with the CM from siRNA-treated valvular interstitial cells reacquired the ability to form capillary-like structures (Fig. 5j). The full lengths of the capillary-like structures were evaluated using the Scion Image computer program (Fig. 5k).

### (Migration assay)

Invasion assay was performed by a known method (Porter, K. E. et al. Simvastatin inhibits human saphenous vein neointima formation via inhibition of smooth muscle cell proliferation and migration. J. Vasc. Surg. 36, 150-7 (2002)) using a modified Boyden chamber that has 8-µm pore filter inserts in 24-well plate (Beckton Dickinson Labware, Franklin Lakes, New Jersey). More specifically, rhVEGF-A was dissolved in EBM2 at 20 ng/mL, and placed in the lower chamber of the Boyden apparatus. NIIH3T3 or valvular interstitial cells (1 x 10⁵ cells/well) were added to the lower chamber and 48 hours later, human coronary artery endothelial cells (5 x 10⁴ cell/well) were seeded in the upper chamber. After 16 hours of incubation, cells that remained bound to the upper surface of the filters were collected using the tip of a cotton-tipped swab, and the number of cells present on the underside of the filter was counted using a light microscope. The assay was performed five times and the results were averaged.

Results obtained by using the modified Boyden chamber revealed that ChM-I also inhibited the migration ability of human coronary artery endothelial cells. In comparison with NIH3T3 cells, human coronary artery endothelial cells cocultured with valvular interstitial cells lost their ability to migrate to the underside of the membrane (Figs. 5l and m). When valvular interstitial cells were treated with chm-I-specific siRNA, the migration ability of human coronary artery endothelial cells recovered partially (Fig. 5o). These results suggest that ChM-I plays an important role as a chemotactic factor inhibitor in cardiac valves.

### (Annexin V-FITC / propidium iodide assay)

Morphological changes of human coronary artery endothelial cells after treatment with valvular interstitial cell culture supernatant suggest that apoptosis is partially involved. To examine whether ChM-I can induce apoptosis, effects of the CM of valvular interstitial cells on apoptosis in human coronary artery endothelial cells were determined using the Annexin V / propidium iodide assay (BioVision Inc., Mountain View, California). After the treatment, the cells were labeled with Annexin V-FITC, and fluorescent cells were counted in six randomly selected visual fields using a Nikon Diaphot microscope (excitation at 488 nm, emission at 530 nm).

The result of positive Annexin V-FITC staining indicated induction of apoptosis (Figs. 5r and t). The fluorescent cells were both propidium iodide-positive (labeling pattern of early apoptosis) and negative (labeling pattern of late apoptosis). Human coronary artery endothelial cells treated with CM from NIH3T3 cells were Annexin V-FITC-negative (Figs. 5q and r). Annexin V-FITC-positive fluorescent cells were counted at high magnification (Fig. 5u). These findings suggest that VIC-derived ChM-I protects cardiac valves from endothelial infiltration and vascular formation.

### (Statistical Analysis)

The values are shown as the mean ± SEM. When comparing two mean values, statistical significance was evaluated using an unpaired Student's t-test. Multiple comparisons of more than three groups were made using ANOVA. A value of p < 0.05 was considered significant.

### [Example 12] Induction of VEGF-A expression, angiogenesis, thickening and calcification in cardiac valves due to the disruption of chm-I gene

To elucidate the function of ChM-I *in vivo,* the cardiac valves of chm-I^{-/-} mice were examined. At eight weeks old, histological differences between chm-I^{-/-} and wildtype mice could not be observed. In old age (90.2 ± 7.4 weeks old), the cardiac valves of chm-I^{+/+} mice were significantly thicker and the density of valvular interstitial cells was more sparse than in the age-matched wildtype mice (Fig. 6). The cardiac valves of chm-I^{-/-} mice showed that the ChM-I and VEGF-A expression patterns are consistent with the diseased state (ChM-I-negative and VEGF-A-positive), and newly formed capillary-like structures were present. Cells that formed capillary-like structures were endothelial cells since they were anti-vWF antibody-positive. Calcium deposition in the cardiac valves of chm-I^{-/-} mice were detected by von Kossa staining (data not shown), and the presence of inflammatory cells were confirmed by MAC-1-positive staining. Formation of new blood vessels and infiltration of inflammatory cells indicated sclerotic process in the heart valves of aged chm-I^{-/-} mice. The age-matched wildtype mice did not show such characteristics. HE staining and immunostaining with VEGF-A and v-WF showed that there were very few blood vessels in the heart valves of age-matched chm-I^{+/+} mice. Capillary structures in the three lobules of the aortic valves were counted after HE staining for sections sampled every 20 µm. The number of capillaries in chm-I^{-/-} mice (n = 7) was 16.7 times greater than in chm-I^{+/+} mice (n = 7).

### [Example 13] Development of early-phase aortic stenosis in chm-I^{-/-} mice detected by echocardiography

Transthoracic echocardiography was carried out with a Sonos 1000 echocardiography unit (Hewlett-Packard) equipped with a 10-MHz linear-array transducer. Two-dimensional (2D) images of the hearts were taken in the color Doppler mode at the aortic valve long- and short-axis views.

Echocardiography revealed the bright echogenic aortic valve that oscillated with slight acoustic shadow, suggesting thickening or calcification of the cardiac valve (Fig. 7). Color Doppler examination showed a mosaic turbulent jet distal to AV. No echogenic object or turbulent jet was observed in the hearts of chm-I^{+/+} mice. There were no significant differences between chm-I^{+/+} and chm-I^{-/-} mice in the left ventricle diameter, left ventricular wall thickness, brightness of mitral valves (MV), and turbulent flow in the area of MV

### Industrial Applicability

The underlying mechanism of cardiac valve degeneration has hardly been elucidated despite its clinical importance in heart diseases and intensive pathological analyses in this field. The present inventors succeeded in verifying the functional mechanism of ChM-I, which is an anti-angiogenic factor that serves to prevent valvular heart diseases by maintaining avascularity in cardiac valves.

More specifically, the present inventors showed the following findings:
(i) ChM-I was expressed tissue specifically in the atrioventricular cushions and cardiac outflow tract at E9.5, in the ventricular myocardium at E10.0, and in the cardiac valves from late-stage embryogenesis to adults;
(ii) in ApoE^{-/-} mice, as well as in patients with infective pericarditis, rheumatic valvular heart disease, and atherosclerosis, ChM-I expression decreased remarkably, while VEGF-A expression was enhanced;
(iii) ChM-I secreted by VICs plays an important role in the suppression of *in vitro* angiogenesis by human coronary artery endothelial cells;
(iv) capillary-like structures and the number of inflammatory cells representing valvular heart disease increased in aged chm-I^{-/-} mice;
(v) transthoracic echocardiographic analyses of aged chm-I^{-/-} mice showed thickening and calcification of the aortic valve as well as turbulent flow from the aortic valve, and suggested initial changes in aortic stenosis; and such.

ChM-I is expressed in the cartilages, eyes, and thymus of various species including humans (Hiraki, Y. et al., Eur. J. Biochem. 260, 869-78 (1999)), rabbits (Shukunami, C. & Hiraki, Y., Biochem. Biophys. Res. Commun. 249, 885-90 (1998)), mice (Shukunami, C. et al., Int. J. Dev. Biol. 43, 39-49 (1999)), chickens (Shukunami, C. et al., FEBS Lett. 456, 165-70 (1999); Dietz, U. H. et al., Dev. Dyn. 216, 233-43 (1999)), cattle (Hiraki, Y. et al., Biochem. Biophys. Res. Commun. 175, 971-977 (1991)), and zebrafish (Sachdev, S. W. et al., Mech. Dev. 105, 157-62 (2001)). In the present application, ChM-I expression in normal and diseased cardiac valves was examined, and it revealed that ChM-I expression is maintained throughout life in normal cardiac valves, whereas this expression is not maintained in diseased cardiac valves. This finding strongly suggests that ChM-I plays an important role in maintaining cardiac valve function.

Fariba has reported that expression of endostatin, an anti-angiogenic factor derived from an internal fragment of collagen type XVIII, was enhanced in aortic valves in a diseased state, but not under normal conditions (Chalajour, F. et al., Exp. Cell Res. 298, 455-64 (2004)). The present application is the first that reports an anti-angiogenic factor expressed in cardiac valves under physiological conditions suppresses angiogenesis. Cardiac valves are flow-regulating tissues in a dynamic chambered pump; therefore, they are subjected to mechanical stress and damage in the endothelial cells that line the outer layer of the valves.

Identification of ChM-I as a factor that protects cardiac valves from inflammation due to mechanical damage and vasculogenesis by the present inventors is very valuable. To confirm the hypothesis that ChM-I functions as a protective factor against valvular heart disease, the present inventors analyzed the ChM-I expression profile in cardiac valves under diseased state, in an *in vitro* model, and in chm-I^{-/-} mice serving as an *in vivo* valvular heart disease model. The present inventors showed that ChM-I expression was dramatically down-regulated in diseased valves, but in contrast, VEGF-A was remarkably up-regulated. Such expression patterns of ChM-I and VEGF-A can be described by several mechanisms.

First of all, an upstream signal may control gene switching between angiogenic and anti-angiogenic factors. In support of this mechanism, Takeda showed that in cbfal^{-/-} mice, change in the expression of Cbfa1, an important transcription factor mediating endochondral ossification in cartilages, was induced through coordination between angiogenesis stimulation in chondrocytes (up-regulation of VEGF-A) and suppression of angiogenesis (down-regulation of ChM-I) (Takeda, S. et al., Genes Dev. 15,467-81 (2001)). Rajamannan showed that Cbfa1 expression was up-regulated in the aortic valve in atherosclerosis (Akiyama, H. et al., Proc. Natl. Acad. Sci. USA 101, 6502-7 (2004)). The up-regulated Cbfa1 in the diseased valve may induce ChM-I and VEGF-A expression.

Secondly, acute inflammation of cardiac valves in rheumatic fever or infective endocarditis, excessive mechanical stress on valves such as bicuspid aortic valve in hypertension, and chronic inflammation observed in atherosclerosis may induce loss of valvular interstitial cells. Decrease in valvular interstitial cells may cause decrease in the level of ChM-I which is produced by diseased valves, and subsequently, this may cause infiltration of VEGF-A-expressing cells. Similarly, a combination of such two mechanisms may explain the down-regulation of ChM-I and the up-regulation of VEGF-A expression.

Western blot analysis of cardiac valves identified a 25-kDa protein, and the mature glycosylated secretory form of ChM-I was found in cartilages. *In vitro* immunostaining experiments strongly suggest that ChM-I expressed in cardiac valves is a mature secretory form of the protein bound to a matrix protein in valvular interstitial cells.

The culture supernatant of valvular interstitial cells suppressed tube formation and migration of human coronary artery endothelial cells *in vitro,* and induced apoptosis of these cells. The suppression of anti-angiogenic activity of the culture supernatant of valvular interstitial cells by ChM-I-specific siRNA suggests that ChM-I is an important anti-angiogenic factor in cardiac valves. This incomplete suppression of anti-angiogenic activity suggests that valvular interstitial cells may be secreting other anti-angiogenic factors similar to the factors identified in the eyes. In the eyes, anti-angiogenic factors endostatin and PEDF (pigment epithelium-derived factor) as well as ChM-I are expressed (Dawson, D. W. et al., Science 285, 245-8 (1999)).

Angiogenesis and infiltration of inflammatory cells were observed in the cardiac valves of aged chm-I^{-/-} mice. By using echocardiography, the presence of calcified and thickened aortic valves in these mice was proved. Further, mosaic and turbulent flow distal to the aortic valve was observed. These findings provide a proof that chm-I^{-/-} mice show initial changes related to aortic stenosis. Pathological changes in atherosclerosis-related activities including infiltration of macrophages, presence of VEGF-A positive cells, and calcification indicate changes in cardiac valves caused by ordinary mechanical stress in the absence of protective effects of anti-angiogenic factors.

Nakamichi discovered that at the age of 12 weeks, chm-I^{-/-} mice had significant enhancement of bone mineral density, and significant angiogenesis in the aortic valve was observed in aged chm-I^{-/-} mice in the present invention. Recently, Docheva has reported that mice lacking both chm-I and its homolog, tenomodulin, did not show changes in angiogenesis after oxygen-induced retinosis (Docheva, D. et al., Mol. Cell Biol. 25, 699-705 (2005)). The difference between cardiac valves and retina may be explained by the presence of anti-angiogenic factors which are not present in cardiac valves in retina. Avascularity observed in the cardiac valves of young adult knockout mice suggests that angiogenesis in aged chm-I^{-/-} mice is not a result of the development of damaged blood vessels in their hearts, but is due to regressive changes in the valves induced by age and inflammation.

The studies carried out so far have shown that (i) expression of angiogenic factors and vasculogenesis take place in valvular heart diseases (VHD) (Soini, Y. et al., Hum. Pathol. 34, 756-63 (2003); Chalajour, F. et al., Exp. Cell Res. 298, 455-64 (2004); Shworak, N. W., Curr. Opin. Cardiol. 19, 140-6 (2004); Mohler, E. R., 3rd et al., Circulation 103, 1522-8 (2001); Mazzone, A. et al., J. Am. Coll. Cardiol. 43, 1670-6 (2004)); (ii) progression of atheroma plaques is related to angiogenesis (O'Brien, K. D. et al., Circulation 93, 672-82 (1996); Moulton, K. S. et al., Proc. Natl. Acad. Sci. USA 100, 4736-41 (2003)); (iii) initial lesion of degenerative valvular heart diseases is an active inflammatory process with certain similarity to atherosclerosis (Mohler, E. R., 3rd et al., Circulation 103, 1522-8 (2001); Otto, C. M. et al., Circulation 90, 844-53 (1994); O'Brien, K. D. et al., Circulation 92, 2163-8 (1995); Agmon, Y. et al., J. Am. Coll. Cardiol. 38, 827-34 (2001); O'Brien, K. D. et al., Circulation 106, 2224-30 (2002); Pohle, K. et al., Mayo. Clin. Proc. 79, 1242-6 (2004)); and (iv) endothelial cells in valvular heart diseases show significant increase in angiogenic ability (Chalajour, F. et al., Exp. Cell. Res. 298, 455-64 (2004)). These findings suggest that angiogenesis in the cardiac valves with valvular heart disease causes the progress of cirrhogenic changes.

Considering the above in combination, findings obtained by the present invention strongly support the mechanism that anti-angiogenic factors protect cardiac valves from the occurrence of angiogenesis and dystrophic changes.

Furthermore, in the artificial heart valves provided by the present inventors, angiogenesis into the valvular tissues is suppressed, and this is considered to contribute to their long-term functional maintenance.

## Claims

1. An angiogenesis-suppressing agent comprising as an active ingredient any one of:
(a) a chondromodulin-I protein;
(b) a protein functionally equivalent to a chondromodulin-I protein, which comprises an amino acid sequence with one or more amino acid deletions, substitutions, or additions in the amino acid sequence of the chondromodulin-I protein; and
(c) a DNA encoding the protein of (a) or (b).

2. The angiogenesis-suppressing agent of claim 1, which has an effect of suppressing angiogenesis in cardiac valves.

3. The angiogenesis-suppressing agent of claim 1, which has an effect of suppressing angiogenesis in retina.

4. A therapeutic agent for angiogenesis-related disease comprising as an active ingredient any one of:
(a) a chondromodulin-I protein;
(b) a protein functionally equivalent to a chondromodulin-I protein, which comprises an amino acid sequence with one or more amino acid deletions, substitutions, or additions in the amino acid sequence of the chondromodulin-I protein; and
(c) a DNA encoding the protein of (a) or (b).

5. A therapeutic agent for angiogenesis-related disease comprising as an active ingredient a chondromodulin-I protein expression-activating substance or a chondromodulin-I protein function-activating substance.

6. The therapeutic agent for angiogenesis-related disease of claim 4 or 5, wherein the angiogenesis-related disease is a disease caused by angiogenesis in cardiac valves.

7. The therapeutic agent for angiogenesis-related disease of claim 4 or 5, wherein the angiogenesis-related disease is a disease caused by angiogenesis in retina.

8. The therapeutic agent for angiogenesis-related disease of claim 4 or 5, wherein the angiogenesis-related disease is a disease selected from the group consisting of valvular heart disease, infective endocarditis, rheumatic heart disease, atherosclerosis, and retinosis.

9. The pharmaceutical agent of any one of claims 1 to 8, wherein the chondromodulin-I protein is a protein comprising the amino acid sequence of SEQ ID NO: 2.

10. A gene knockout non-human animal, wherein expression of a chondromodulin-I gene is artificially suppressed.

11. The gene knockout non-human animal of claim 10, which has an abnormality in its cardiac valve.

12. The gene knockout non-human animal of claim 10 or 11, which is used to screen for a therapeutic agent for angiogenesis-related disease.

13. A method of screening for a therapeutic agent for angiogenesis-related disease, which comprises selecting a compound that activates expression or function of a chondromodulin-I protein.

14. A method of screening for a therapeutic agent for angiogenesis-related disease, which comprises the steps of:
(a) contacting a test compound with cells expressing a chondromodulin-I protein;
(b) measuring expression level of the chondromodulin-I protein in said cells; and
(c) selecting a compound that increases the expression level compared with that measured in the absence of the test compound.

15. A method of screening for a therapeutic agent for angiogenesis-related disease, which comprises the steps of:
(a) contacting a test compound with cells or an extract solution of cells comprising a DNA structured such that a reporter gene is operably linked to the transcriptional regulatory region of a chondromodulin-I gene;
(b) measuring expression level of said reporter gene; and
(c) selecting a compound that increases the expression level compared with that measured in the absence of the test compound.

16. A method of screening for a therapeutic agent for angiogenesis-related disease, which comprises the steps of:
(a) contacting a test compound with a chondromodulin-I protein, or cells or an extract solution of cells expressing said protein;
(b) measuring activity of said protein; and
(c) selecting a compound that increases the protein activity compared with that measured in the absence of the test compound.

17. A method of screening for a therapeutic agent for angiogenesis-related disease, which comprises the steps of:
(a) administering a test compound to the gene knockout non-human animal of any one of claims 10 to 12;
(b) measuring the expression level or activity of chondromodulin-I protein in said gene knockout non-human animal; and
(c) selecting a compound that increases the expression level or activity of chondromodulin-I protein compared with when the test compound is not administered.

18. The screening method of claim 17, wherein the expression level or activity of said protein in the cardiac valve or eye of said non-human animal is measured.

19. A method of screening for a therapeutic agent for angiogenesis-related disease, which comprises the steps of:
(a) administering a test compound to the gene knockout non-human animal of any one of claims 10 to 12;
(b) observing the cardiac valve or retina in said gene knockout non-human animal; and
(c) selecting a compound that normalizes the cardiac valve or retina compared with when the test compound is not administered.

20. The screening method of any one of claims 13 to 19, wherein the angiogenesis-related disease is valvular heart disease, infective endocarditis, rheumatic heart disease, atherosclerosis, or retinosis.

21. An artificial heart valve comprising as major components:
(a) cells expressing a chondromodulin-I gene; and
(b) a decellularized valve or biodegradable high-molecular compound.

22. An artificial heart valve produced by the steps of:
(a) culturing and growing cells that express a chondromodulin-I gene;
(b) seeding the cells of (a) onto the surface of a decellularized valve or biodegradable high-molecular compound; and
(c) culturing until the cells expressing the chondromodulin-I gene fill the pore spaces of the decellularized valve or biodegradable high-molecular compound of (b).
